# EUROPEAN PATENT SPECIFICATION

(11) **EP 3 817 651 B1**
(45) Date of publication and mention of the grant of the patent: **18.09.2024**
(21) Application number: 19830800.9
(22) Date of filing: 05.07.2019
(51) Int. Cl.: A61B 5/00, A61B 5/145, A61B 5/1455

(54) **APPARATUS AND METHOD FOR MONITORING BRAIN ACTIVITY**
VORRICHTUNG UND VERFAHREN ZUR ÜBERWACHUNG DER GEHIRNAKTIVITÄT
APPAREIL ET PROCÉDÉ POUR SURVEILLER L'ACTIVITÉ CÉRÉBRALE

(30) Priority: 06.07.2018 US 201862694447 P
(43) Date of publication of application: 12.05.2021
(73) Proprietor: Axem Neurotechnology Inc., Dartmouth, Nova Scotia B3B 1V7 (CA)
(72) Inventor: FRIESEN, Christopher, Halifax, Nova Scotia B3J 2Y9 (CA); INGRAM, Tony Joseph Gerald, Dartmouth, Nova Scotia B3A 2G7 (CA); HAMILTON, Eric Anthony, Halifax, Nova Scotia B3J 1Y3 (CA); LAWRENCE, Michael, Halifax, Nova Scotia B3K 0H2 (CA); LEUSCHEN, Karl Ryland, Brookside, Nova Scotia B3T 1R4 (CA); SMITH, Megan Melissa, Nine Mile Road, Nova Scotia B2S 2W3 (CA); HALIBURTON, Luke Murphy, Antigonish, Nova Scotia B2G 2L4 (CA); MURPHY, Chad Douglas, Porters Lake, Nova Scotia B3E 0A1 (CA)
(74) Representative: Studio Torta S.p.A.
(86) International application number: PCT/CA2019/050935
(87) International publication number: WO 2020/006647

(56) References cited:
- WO-A1-2009/134674
- WO-A1-2017/145723
- US-A1- 2010 041 963
- US-A1- 2010 317 949
- US-A1- 2013 303 874
- US-A1- 2016 174 857
- US-A1- 2017 231 501
- US-A1- 2017 231 501
- US-A1- 2017 319 116

## Description

### BACKGROUND

### 1. Field

This disclosure relates generally to brain activity monitoring and more particularly to brain activity monitoring using infrared light.

### 2. Description of Related Art

Near-infrared Spectroscopy may be used to measure brain activity in the motor cortex by measuring relative changes in oxygen concentration in the brain. Brain activity requires oxygen to use energy, which is known as the hemodynamic response and is the basis for many brain imaging technologies. When a user moves their left hand, the concentration of oxygen will increase in the right motor cortex in the area that controls the hand. The more muscle recruitment and the more complex the movement, the greater the oxygen change.

Individuals with an acquired brain injury (such as a stroke) often have mobility impairments, requiring intensive physical rehabilitation. Rehabilitation promotes recovery by leveraging neuroplasticity (i.e. the brain's ability to change). Brain activity metrics may be used to predict recovery, track progress, and compare the effects of different exercises, potentially allowing clinicians to better tailor therapy to individual patients. There remains a need for brain activity monitoring methods and apparatus.

Patent application US2017/231501 relate to measuring brain activity using diffuse optical tomography and, more specifically, to the measuring of brain activity using super-pixel detection with wearable diffuse optical tomography.

Patent application WO2017/145723 relates to a biological measurement apparatus that measures a state of a living body by a measurement section having a light source section and a detection section, a measurement section of the biometric measurement apparatus, and a protection cap attached to the measurement section.

Patent application WO2009/134674 relates to an optode and an electrode- optode cap having multiple optodes for use in diffuse-optical imaging, and in particular in Near-InfraRed Diffuse-Optical Tomographic Functional Neuro Imaging (NIR-DOTFNI) and correlation of NIR-DOTFNI with electroencephalography.

Patent application US2013/303874 pertains to the fields of electroencephalography, diffuse optical imaging, and functional neuroimaging.

Patent application US2016/174857 relates to an apparatus, system, and method for the detection of the occurrence of cardiac arrest in a human subject followed by the prompt issuance of an audible alarm, as well as a vibration alert and cellular phone transmission of synthesized speech alerts and location of human subject to pre-determined list or alternative list of phone numbers according to a precise Global Positioning Satellite (GPS) derived location of subject. The verbal alerts would be issued to one or more persons and emergency medical services that are capable of providing life saving interventions (referred to hereinafter as "first responders").

However, the above-mentioned issues remain unsolved.

### SUMMARY

The present invention relates to an apparatus for monitoring brain activity of a user as defined in the annexed claims.

In an aspect of the present disclosure, the apparatus includes a plurality of spatially separated emitters operable to generate infrared radiation. The apparatus also includes a plurality of spatially separated infrared radiation detectors, and a plurality of light pipes urged into contact with the user's scalp, each one of the plurality of emitters and detectors having an associated light pipe operable to couple infrared radiation from the emitter into the scalp or to couple infrared radiation from the scalp to the detector. Each detector is operable to produce a signal representing an intensity of infrared radiation generated by a selectively actuated one of the plurality of emitters and received at the detector after traveling on a path through underlying brain tissue, the signals being received by a controller operably configured to process the signals from each detector to determine changes in blood oxygenation within the brain tissue along the path between the respective emitter and detector, and generate a spatial representation of brain activity within in the user's brain based on the processed signals.

The emitters and detectors are disposed on a headset and the controller may be remotely disposed with respect to the headset and the headset may include a transmitter operable to transmit the signals to the controller for processing.

The apparatus may include a headset controller disposed on the headset and operably configured to control functions of the transmitter, the emitters, and the detectors.

The infrared radiation may include near infrared radiation.

The emitter includes a light emitting diode operably configured to produce the infrared radiation at a plurality of wavelengths selected to cause the detector to produce signals that facilitate determination of a blood oxygenation state of the brain tissue underlying each of the spatially separated emitters and associated detectors, the blood oxygenation state being indicative of local cerebral hemodynamics within the brain tissue and facilitating a determination of neural activity within the user's brain.

The plurality of wavelengths may include at least first and second wavelengths selected to fall on either side of the isobestic point for oxygenation and deoxygenation of blood hemoglobin.

The light emitting diode associated with each of the plurality of emitters is mounted within a headset,
the headset being operable to support the plurality of emitters and plurality of detectors in contact with the user's scalp when worn by the user.

The plurality of emitters includes at least one emitter disposed proximate to one of the plurality of detectors and the detector may be operable to produce a shallow path signal representing an intensity of infrared radiation generated after traveling along a shallow path through scalp and bone tissue between the at least one emitter and the detector, at least one emitter disposed spaced apart from one or more of the plurality of detectors and the one or more detectors are operable to produce a deep path signal representing an intensity of infrared radiation generated after traveling along a deep path through the underlying brain tissue between the at least one emitter and the one or more detectors.

The controller may be operably configured to process the shallow path signals to determine shallow path noise, the shallow path noise being used as a basis for filtering the deep path signal to determine the changes in blood oxygenation within the brain tissue.

The controller may be operably configured to process the shallow path signals to determine shallow path noise, the shallow path noise being used as a basis for filtering the deep path signal to determine the changes in blood oxygenation within the brain tissue.

The controller may be operably configured to process the signals by aligning a phase of each of the shallow path signals and deep path signals based on a physiological process component in the signals, performing a principle component analysis on the shallow path signals to determine contamination components associated with physiological processes other than changes in blood oxygenation within the brain tissue, and removing the contamination components from the deep path signals to provide signals representing changes in blood oxygenation within the brain tissue from which the effects of other physiological processes have been filtered.

Performing the principle component analysis may include filtering the shallow path signals to separate the shallow path signals into slow-cycling signals associated with slow-cycling physiological processes and fast-cycling signals associated with fast-cycling physiological processes and performing principle component analysis on each of the shallow path signals, the slow-cycling signals and the fast-cycling signals.

The controller may be operably configured to, prior to performing the principle component analysis, process the phase aligned shallow path signals to generate signals representing oxygenation and deoxygenation of blood hemoglobin, and take a first derivative of the signals representing oxygenation and deoxygenation of blood hemoglobin.

The controller may be operably configured to activate selected emitters and detectors to generate signals associated with different paths of travel of the infrared radiation through the brain tissue.

Each light pipe includes a low durometer material that is optically transmissive at wavelengths associated with the infrared radiation, the low durometer material facilitating comfortable optical contact with the scalp of the user.

The light pipe material has a durometer in a range of between about Shore A durometer 30 and about Shore A durometer 90.

The length of each light pipe may be between about 7 millimeters and 15 millimeters.

Each of the plurality of emitters and detectors are mounted on a headset that conforms to the scalp of the user and a length of at least about 7 mm of the light pipe may protrude outwardly from a surface of the headset.

Each light pipe includes a coupling surface for coupling infrared radiation between the light pipe and the emitter or detector, a distal lens operably configured to contact the scalp and direct infrared radiation to or from the light pipe, and a guide portion extending between the coupling surface and the distal lens.

The apparatus includes a sheath surrounding at least a portion of the guide portion of each light pipe, the sheath being operably configured to reduce infrared radiation leakage from the guide portion of the light pipe.

The sheath may include an outer surface operably configured to divert the user's hair away from the distal lens when the light pipe is in contact with the scalp.

The guide portion of the light pipe may have a generally cylindrical shape and may have a diameter selected to cause total internal reflection of infrared radiation incident at inner surfaces of the guide portion.

The coupling surface of the light pipe may be operably configured to directly contact a radiating surface of the emitter or a radiation receiving surface of the detector for coupling infrared radiation between the light pipe and the detector.

A cross sectional area of the guide portion may be smaller than a cross sectional area of the coupling surface and the light pipe may further include a tapered transition between the coupling surface and the guide portion and a taper angle of the tapered transition may be selected to prevent infrared radiation leakage from the tapered transition, the tapered transition further providing for mounting of the light pipe to the emitter or detector.

The apparatus includes a headset having a plurality of articulated segments, each articulated segment supporting at least one emitter or detector, the articulated segments each being urged toward the scalp of the user to cause contact between the associated light pipes of the respective emitters or detectors and the scalp.

Each of the plurality of articulated segments may be operably configured to mount a circuit substrate and at least one detector or emitter may be mounted on each circuit substrate.

The apparatus may include a flexible interconnect interconnecting between a headset controller and the plurality of circuit substrates.

The flexible interconnect and the plurality of circuit substrates may be formed as a unitary flexible circuit substrate.

The plurality of detectors are disposed spaced apart along a sprung band having a curvature operable to conform to a corresponding lateral curvature of the user's scalp and urge the plurality of detectors toward the scalp when the band is worn by the user.

The apparatus may further include a plurality of articulated segments disposed forwardly or rearwardly with respect to the sprung band, each articulated segment including at least one emitter and being urged toward the scalp when the band is worn by the user.

The controller may be operably configured to monitor the signal level produced at each detector and to control a level of infrared radiation produced by the selectively actuated emitter to maintain the intensity within a detection range of the detector.

The controller may be further operably configured to generate display data for display as a graphic user interface (GUI) on a screen in communication with the controller, the GUI including a spatial representation of at least one of the emitters and detectors along with display information indicating whether the signal intensity is within the detection range of the associated detector.

The controller may be operably configured to discontinue the monitoring when the signals received from the detectors no longer meet a coupling criterion indicative of a plurality of the emitters or detectors being coupled to the scalp of the user.

The apparatus includes at least one coupling sensor operably configured to generate a coupling signal indicating a state of coupling between the plurality of light pipes and the user's scalp, and the controller may be operably configured to discontinue the monitoring in response to the coupling signal indicating that a coupling criterion is not being met.

The at least one coupling sensor may include at least one of a capacitive sensor that produces a signal indicative of a proximity of the apparatus to the scalp, an acoustic sensor that produces a signal in response to an ambient sound level, an inertial sensor that produces a signal indicative of movement of the apparatus, or one or more of the detectors, an ambient light component in the signal produced by the one or more detectors may be indicative of the apparatus being removed from the scalp and the detector being subject to ambient light radiation.

The controller may be operably configured to process the signal received by at least one of the detectors to extract a cardiac pulse signal representing a detected heartbeat of the user and to monitor the pulse signal to determine whether coupling between the emitters and detectors and the scalp of the user meets a coupling criterion.

The controller may be operably configured to process the signals by extracting a dominant frequency from the signals that falls within a frequency range based on the user's expected heartbeat frequency range. The controller may be operably configured to discontinue the monitoring when the cardiac pulse signals received from the detectors no longer meet the coupling criterion.

The controller may be operably configured to monitor time variations in blood oxygenation within the brain tissue in a region underlying each detector and selectively actuated emitter and to generate data metrics representing a degree of brain activation in each region.

The controller may be further operably configured to generate display data for display as a graphic user interface (GUI) on a screen in communication with the controller, the GUI including a representation of regions of the user's body that correspond to regions of the user's brain that are indicated by the changes in blood oxygenation within the brain tissue to be actuated.

The controller may include a processor circuit, the processor circuit including a graphic processing unit operably configured to accelerate processing of the signals from each of the plurality of detectors to facilitate near real time presentation of results to the user.

Other aspects and features will become apparent to those ordinarily skilled in the art upon review of the following description of specific disclosed embodiments in conjunction with the accompanying figures.

### BRIEF DESCRIPTION OF THE DRAWINGS

In drawings which illustrate disclosed embodiments,
- Figure **1**: is a perspective view of an apparatus for monitoring brain activity in accordance with a first disclosed embodiment;
- Figure **2**A: is an exploded view of an emitter enclosure of the apparatus shown in Figure **1**;

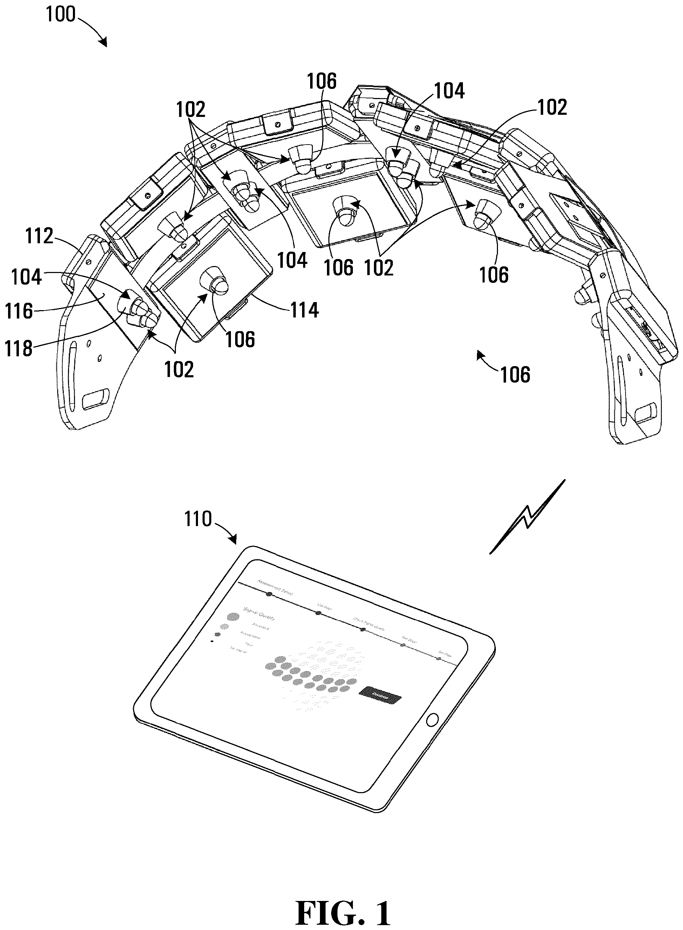

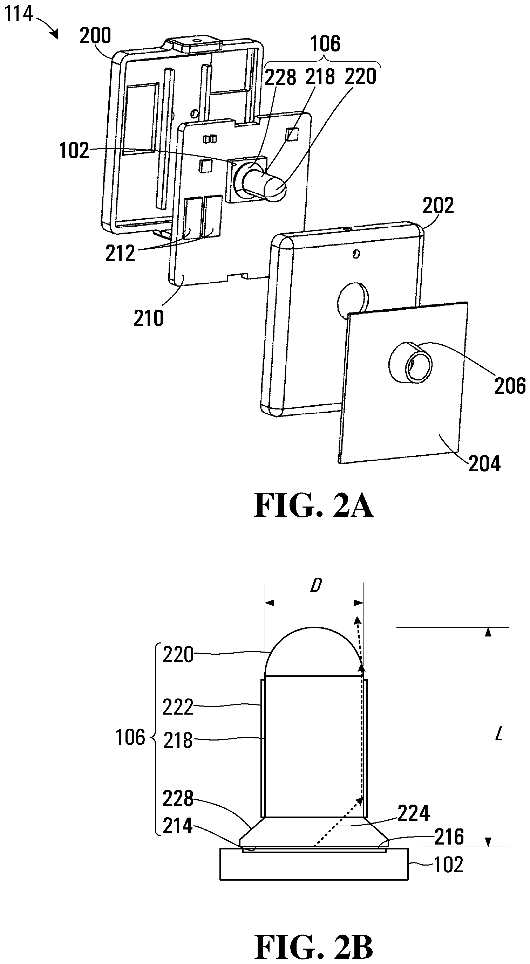

- Figure **2**B: is an elevational view of an emitter and light pipe of the apparatus shown in Figure **1**;

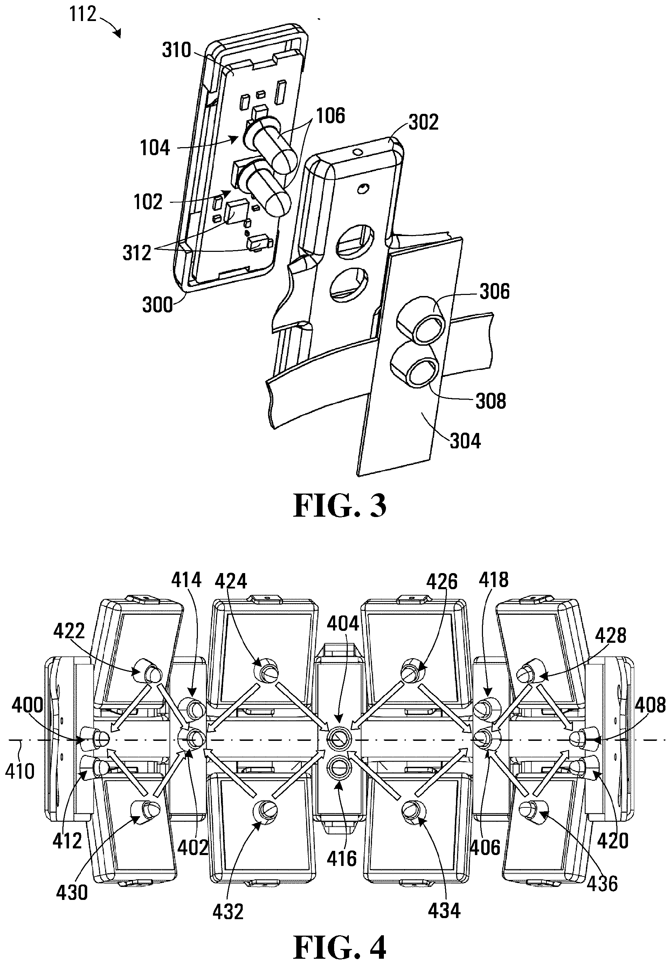

- Figure **3**: is an exploded view of a detector enclosure of the apparatus shown in Figure **1**
- Figure **4**: is a plan view of a headset shown in Figure **1**;

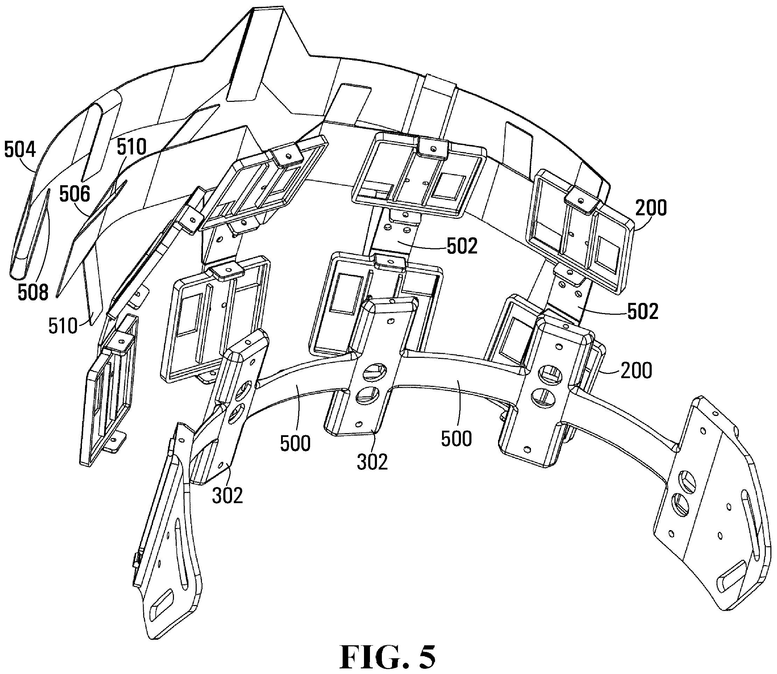

- Figure **5**: is an exploded view of portions of the headset shown in Figure **4**;

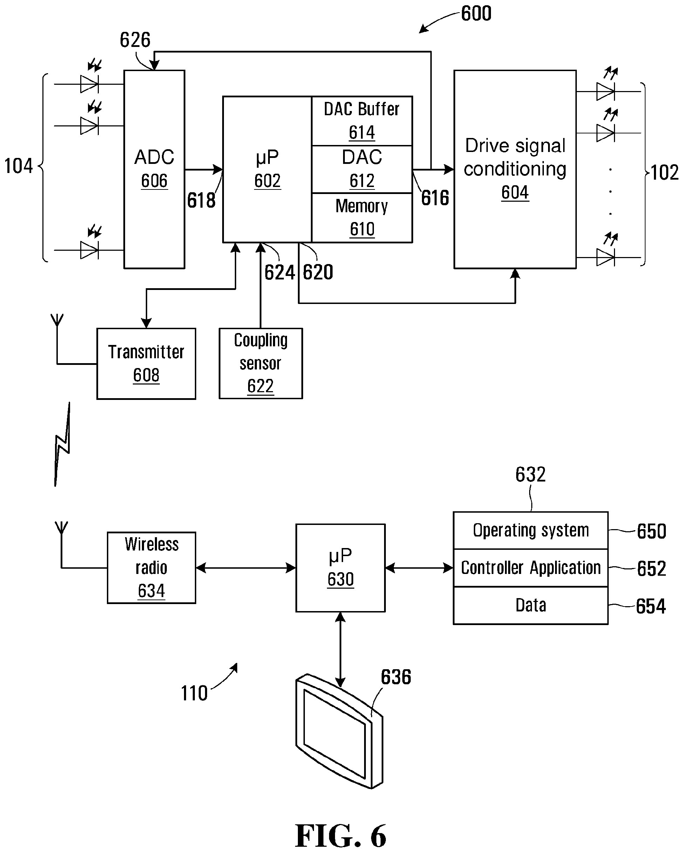

- Figure **6**: is a block diagram of a headset controller and a host controller of the apparatus shown in Figure **1**;

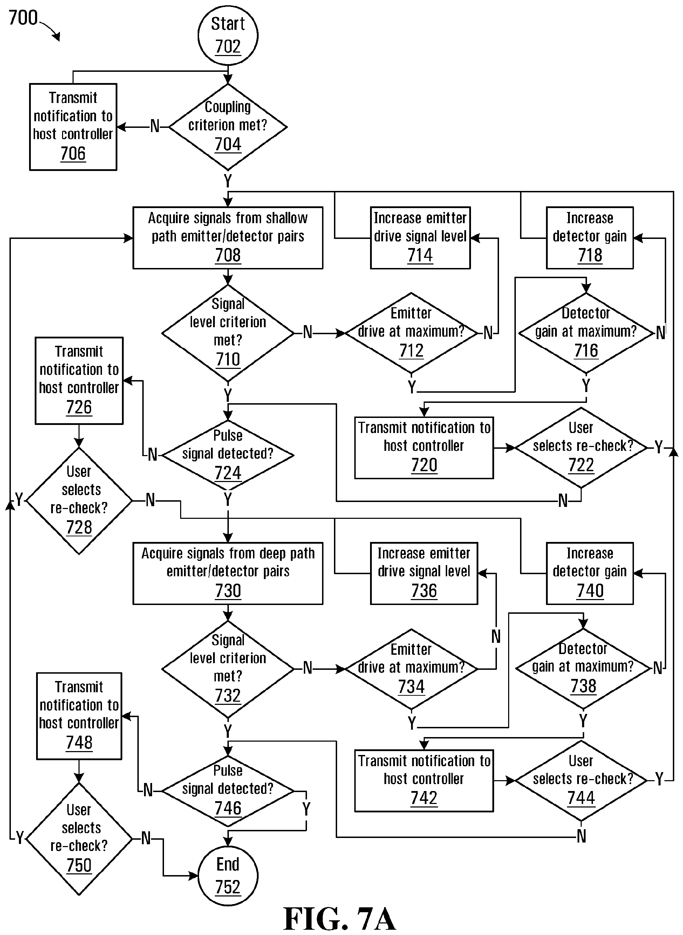

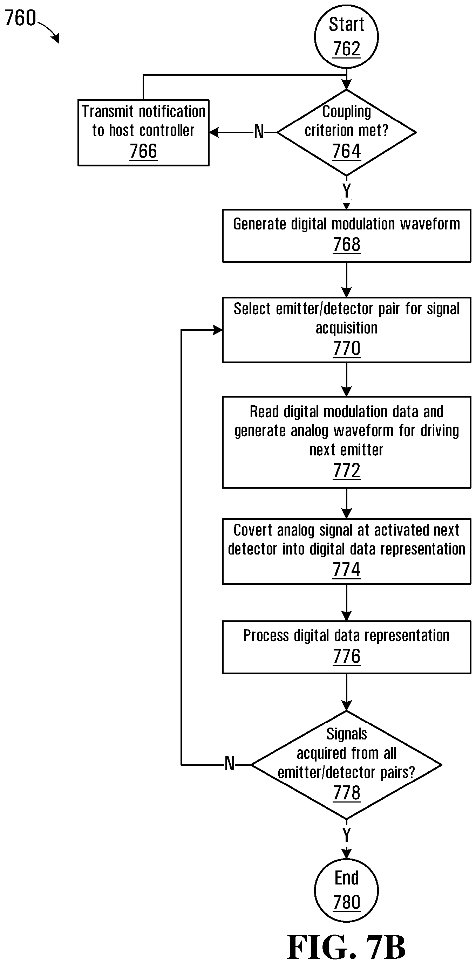

- Figure **7**A: is a process flowchart depicting blocks of code for directing the headset controller shown in Figure **6** to perform a signal calibration process;
- Figure **7**B: is a process flowchart depicting blocks of code for directing the headset controller shown in Figure **6** to acquire signals;

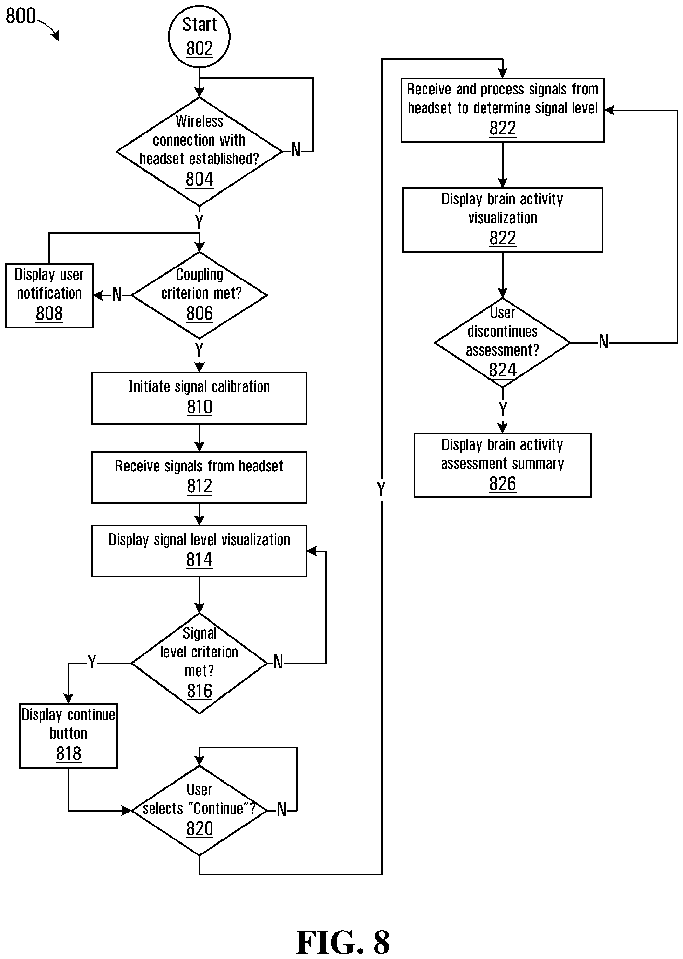

- Figure **8**: is a process flowchart depicting blocks of code for directing a processor circuit of the host controller to perform an assessment session;

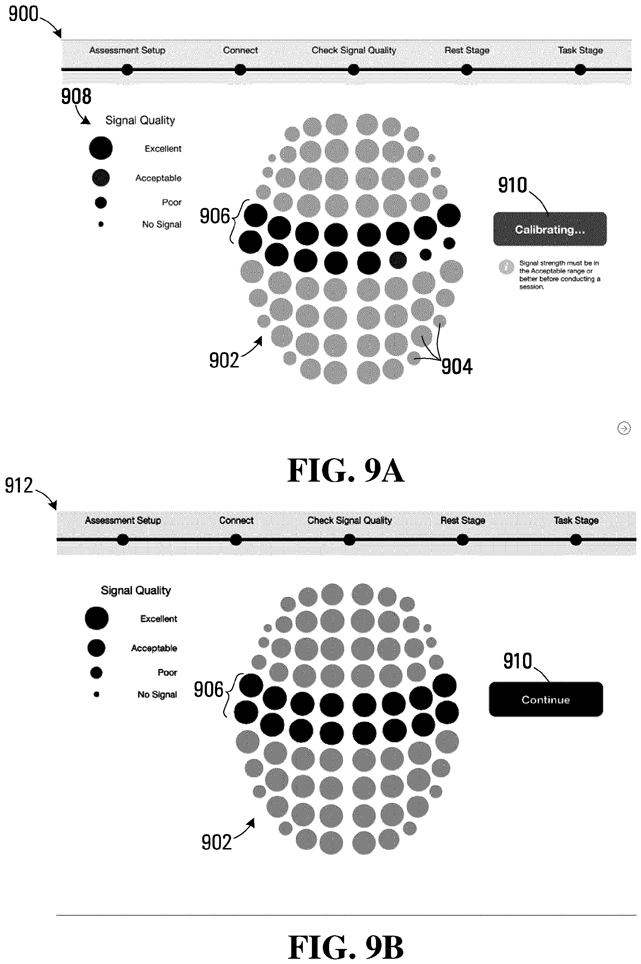

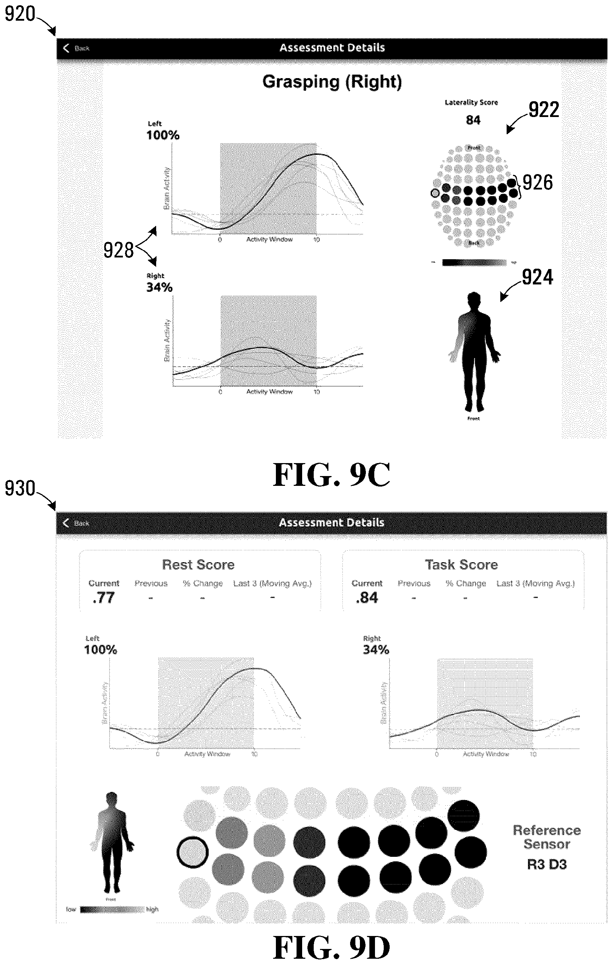

- Figure **9**A-**9**D: are a series of screenshots showing display screens generated and displayed on a display of the host controller; and

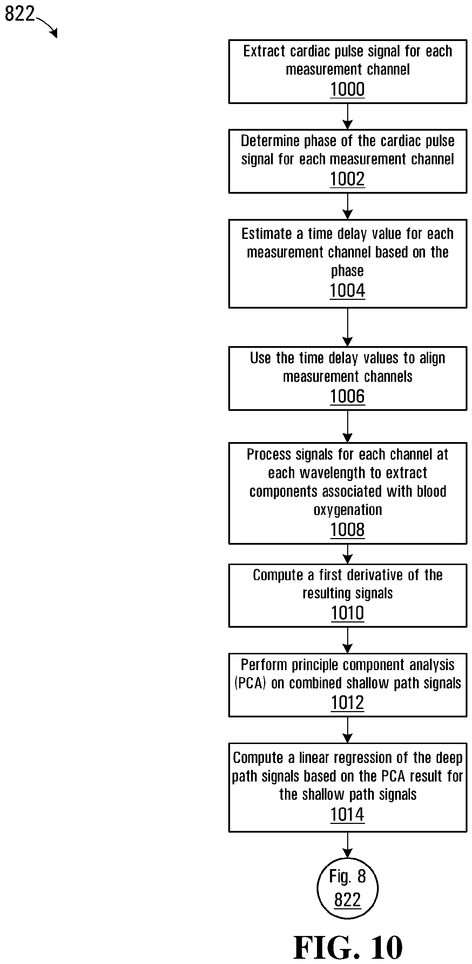

- Figure **10**: is a process flowchart depicting blocks of code for directing the host controller to implement data signal processing functions shown in Figure **8**.\

### DETAILED DESCRIPTION

### System overview

Referring to Figure **1**, an apparatus for monitoring brain activity through a user's scalp according to a first disclosed embodiment is shown generally at **100.** The apparatus **100** includes a plurality of spatially separated near infrared radiation emitters **102** and a plurality of spatially separated near infrared radiation detectors **104.** Each one of the emitters **102** and the detectors **104** have an associated light pipe **106**, which is operable to couple near infrared radiation from the emitter into the user's scalp or to couple near infrared radiation from the scalp to the detector. In this embodiment the emitters **102** are mounted within a headset **108** operable to support the plurality of emitters **102** and plurality of detectors **104** in contact with the user's scalp when the headset is worn by the user such that each of the light pipes **106** contact the user's scalp.

Each detector **104** is operable to produce a signal representing an intensity of near infrared radiation generated by a selectively actuated one of the plurality of emitters **102** and received at the detector after traveling on a path through underlying brain tissue. Near infrared radiation (i.e. near infrared light) has a wavelength generally within a range of about **750** nm (nanometers) to **900** nm and is able to travel through skin, tissue, and bone. The near infrared radiation from each emitter **102** thus penetrates the scalp and skull and travels along a path through respective portions of underlying brain tissue, which reflects the radiation back to one or more of the detectors **104.** By selectively actuating one of the emitters **102** and one of the detectors **104**, the signal produced by the detector may be associated with a region of the user's neurocranium that subtends the emitter and detector. If the emitter **102** and detector **104** are disposed proximate each other, the infrared radiation that reaches the detector will have primarily passed through the superficial scalp and bone tissues and is unlikely to have penetrated brain tissues underlying the bone of the neurocranium. When the emitter **102** and detector **104** are disposed spaced further apart, the infrared radiation that reaches the detector will generally have penetrated the scalp and bone tissues and entered the underlying brain tissue.

In this embodiment, the headset **108** is in wireless communication with a controller **110**, which in this embodiment is implemented using a tablet computing device acting as a host controller. The host controller is thus remotely disposed with respect to the emitters **102** and detectors **104**, and the headset **108** includes a transmitter (not shown) operable to transmit the signals to the controller **110** for processing. The controller **110** receives the signals generated by the detectors **104**, which are processed to determine changes in blood oxygenation within the brain tissue along the path between the respective emitter and detector. Based on the processing of the signals, the controller **110** is able to generate a spatial representation of brain activity within in the user's brain.

In one embodiment each emitter **102** is configured to produce near infrared radiation at two or more wavelengths, which are selected to cause an associated detector to produce signals that facilitate determination of a blood oxygenation state of the underlying brain tissue. For example, first and second wavelengths may be selected that fall on either side of the isobestic point for oxygenation and deoxygenation of blood hemoglobin (Hb) at which deoxy-Hb and oxy-Hb have substantially identical absorption coefficients. For example an emitter that produces wavelengths of 750 nm and 850 nm may be used. In other embodiments the selected wavelengths may fall on the same side of the isobestic point.

The detected intensity of each of the selected wavelengths at the detector **104** is thus indicative of the blood oxygenation state, which in turn is indicative of local cerebral hemodynamics within the brain tissue and facilitates a determination of neural activity within the portion of the user's brain through which the near infrared radiation produced by the emitter has traveled to reach the detector.

In this embodiment the emitters **102** and detectors **104** are disposed on the headset **108** and the controller **110** acts as a host controller, which is remotely disposed with respect to the headset and receives signals transmitted by a transmitter (not shown) on the headset **108.** In this embodiment, the headset **108** may further include a headset controller (not shown) disposed on the headset and operably configured to control signal generation and acquisition by the emitters **102** and the detectors **104** and the transmission of these signals to the host controller **110.**

### Emitters and detectors

In this embodiment the detectors **104** are mounted within a detector enclosure **112**, which also houses some of the emitters **102**. The remaining emitters are each housed in a separate emitter enclosure **114**. One of the emitter enclosures **114** is shown in exploded view in Figure 2A. In this embodiment the emitter enclosure **114** is fabricated in two parts including a rear portion **200** and a front portion **202**. The emitter enclosure **114** also includes a cover **204** having a flanged opening **206**. In other embodiments the detector enclosure **112** may be otherwise configured. The emitter **102** is mounted on a circuit substrate **210** including driver circuitry **212** for driving the emitter. In one embodiment the emitter **102** may be implemented using a light source operably configured to produce the near infrared radiation at each of the first and second wavelengths. Light emitting diode packages having more than one light emitting diode operating at different wavelengths are available from several manufacturers such as Osram Sylvania of Wilmingdon MA, USA.

Referring to Figure 2B, the emitter **102** and light pipe **106** are shown in elevational view. The light pipe **106** has a coupling surface **214** for coupling the near infrared radiation produced by the emitter **102** into the light pipe. In the embodiment shown the coupling surface **214** is in direct contact with a window covering the radiating surface **216** of the light source(s) of the emitter **102** and the near infrared radiation is coupled directly into the light pipe **106.** Minor Fresnel losses may be introduced at an optical interface between the emitter **102** and the coupling surface **214**, however direct contact between the coupling surface and the emitter **102** eliminates the need for adhesives. In the configuration shown, transmission efficiency through the light pipes **106** has been found to be about **76**%. The light pipe **106** further includes a guide portion **218** and a distal lens **220.** In this embodiment the lens **220** is configured as a plano-convex lens, but in other embodiments the lens may have other configurations. The guide portion **218** extends between the coupling surface **214** and the distal lens **220** for guiding the near infrared radiation through the light pipe **106.** The distal lens **220** is operably configured to contact the scalp of the user and direct near infrared radiation from the light pipe **106** through the scalp into the neurocranium of the user.

In the embodiment shown the light pipe **106** also includes a sheath **222** surrounding at least a portion of the guide portion **218** of the light pipe. The sheath **222** is optically absorbent at the wavelengths emitted by the emitter **102** and reduces near infrared radiation leakage from the guide portion **218** of the light pipe **106.** In some embodiments the sheath **222** may include an outer surface that is ribbed or otherwise configured to divert the user's hair away from the distal lens **220** when the light pipe **106** is in contact with the scalp, thereby improving near infrared radiation coupling between the emitters **102** and the scalp.

In the embodiment shown the guide portion **218** has a generally cylindrical shape and has a diameter *D* selected to cause total internal reflection of near infrared radiation or light rays incident at inner surfaces of the guide portion. In one embodiment the light pipe **106** is molded from a liquid silicone rubber material (such as Lumisil LR **7601**/70), which has high optical transmissivity at near infrared radiation wavelengths. Lumisil LR **7601**/70 has a refractive index of 1.41, for which the total internal reflection (TIR) critical angle is about 45.2°. A light ray **224** emitted from the center of the radiating surface **216** of the emitter **102** would thus impinge on the outer surface of the guide portion **218** and would be reflected to travel along the outer surface of the guide. Any light rays from the emitter **102** at an angle greater than 45.2° that impinge on the outer surface of the guide portion **218** would escape from the light pipe **106**, but would be absorbed in the sheath **222**.

In the embodiment shown a cross sectional area of the guide portion **218** is smaller than a cross sectional area of the coupling surface **214** and the light pipe includes a tapered transition **228** between the coupling surface and the guide portion. A taper angle of the tapered portion **228** is selected to prevent near infrared radiation leakage from the tapered transition. In Figure 2B, the light ray **224** at the TIR critical angle first impinges on the outer surface of the guide portion **218** well into the guide portion and the tapered portion **228** thus has negligible effect on near infrared radiation coupling into and guiding through the light pipe **106**. The tapered transition **228** however provides for convenient mounting of the light pipe **106** to the emitter **102** without the use of adhesives. In the embodiment shown in Figure 2A, the flanged opening **206** in the cover **204** has a diameter sized to correspond to the diameter *D* of the guide portion **218.** The guide portion **218** is thus sized to permit the guide portion **218** to protrude through the opening flanged opening **206**, while retaining and urging the tapered portion **228** into close contact with the radiating surface **216** of the emitter **102**. The light pipe **106** may have a length of between about **7** mm and about **15** mm in one embodiment. In one embodiment, a length of at least about 7 mm (millimeters) of the light pipe **106** protrudes outwardly from a surface of the headset **108** at the flanged opening **206** in the cover **204.**

In one embodiment the guide portion **218** of the light pipe **106** has a diameter of about 4 mm, the tapered portion **228** has a diameter of about 6 mm at the coupling surface **214**, and the light pipe has an overall length *L* of about 9 mm. The plurality of emitters **102** may each be configured generally as shown in Figure 2A and 2B.

One of the detector enclosures **112** is shown in exploded view in Figure **3****.** Referring to Figure **3**, the detector enclosure **112** is fabricated in two parts including a rear portion **300** and a front portion **302.** The rear portion **300** and front portion are fabricated as part of a unitary assembly that will be described in more detail below. The emitter enclosure **114** also includes a cover **304** having a pair of flanged openings **306** and **308.** The detector **104** and emitter **102** are both mounted on a circuit substrate **310**, which includes circuitry **312** for monitoring the detector.

In this embodiment the emitter **102** is disposed proximate the detector **104** for use as a shallow path emitter. The configuration of the shallow path emitter **102** is generally similar to the configuration described above in connection with Figures 2A and 2B. The detector **104** may be implemented using a photodiode that is responsive to the selected emitter wavelengths. An example of a suitable photodiode is the S9674 Silicon photodiode available from Hamamatsu Photonics K.K., Japan, which has high responsivity in the wavelengths region around 800 nm and a 2 mm x 2 mm photosensitive near infrared radiation receiving area. For the detector **104**, the light pipe **106** may be configured substantially as shown in Figure 2A, except that the distal lens **220** captures near infrared radiation at the point of contact with the user's scalp and directs the radiation through the light pipe to the coupling surface **214** for illuminating the detector photosensitive area. The coupling surface **214** of the light pipe **106** may directly contact the near infrared radiation receiving surface of the photodetector, as in the case of the emitters **102**.

As disclosed above, in one embodiment the light pipe **106** is molded from a liquid silicone rubber material such as Lumisil LR **7601**/70, which is a relatively compliant material having a Shore A durometer of **70**. The material is biocompatible for skin contact for a period of time that the headset would usually be worn by a user during a brain activity assessment session. The material also has good resistance to environmental and other contaminants. The low durometer of the light pipes **106** facilitates comfortable optical contact with the scalp of the user. The inventors have found that a light pipe material having a durometer in a range of between about Shore A durometer **45** and about Shore A durometer **70** provides an acceptable level of comfort and transmittance of near infrared radiation. However, in some embodiments even more compliant materials having Shore A durometer as low as **30** may be used. Less compressible materials having Shore A durometer as high as **95** may also provide comfort for the user.

The headset **108** of Figure **1** is shown in a view in Figure **4** in which all of the emitters **102** and detectors **102** are visible. Referring to Figure **4**, five of the detectors **104** (numbered **400** - **408** in Figure **4**) are aligned along a centrally disposed band that extends laterally over and conforms to the user's crown such that the detectors are aligned along a frontal plane **410** (extending into the page) through the user's neurocranium when the headset is worn by the user. Of the five detectors **400** - **408**, detector **404** is medially disposed while the remaining detectors are laterally spaced apart from the medial detector.

The emitters **102** include shallow path emitters (labeled as **412** - **420** in Figure **4**) disposed proximate to each one of the detectors **400 - 408.** Four emitters **422 - 428** are disposed spaced rearwardly with respect to the frontal plane **410** and laterally offset from the detectors **400 - 408** such that each of the emitters is aligned between two of the detectors. Four emitters **430 - 436** are disposed spaced forwardly with respect to the frontal plane **410** and also laterally offset from the detectors **400 - 408.** Each of the emitters **412** - **436** and the detectors **400 - 408** may be independently and sequentially actuated. As such, the emitters and detectors may be actuated in sequence such that a single emitter **102** is actuated to couple near infrared radiation through the scalp while a single detector **104** is simultaneously monitored to receive the near infrared radiation after traveling through the underlying brain tissue. In this embodiment, since there are only five detectors and thirteen emitters, a detector may be paired with different emitters to receive signals traveling along different paths through the underlying brain tissues. For example, the detector **402** may be sequentially paired with the emitters **414**, **422**, **424**, **430**, and **432.** In other embodiments, more than one pair of emitter/detector pairs may be activated at the same time if the pairs are spaced apart or modulated at different frequencies to avoid signal interference. Additionally more than one of the shallow path emitters may be activated together with their associated proximate detectors, since the shallow path of the near infrared radiation is less likely to cause interference between signals produced by different emitter/detector pairs.

Each of the shallow path emitters **412** - **420** is disposed proximate to respective detectors **400** - **408**. In one embodiment the spacing between the shallow path emitters **412** - **420** and the respective detectors **400** - **408** is about 8 mm center-to-center. Near infrared radiation that reaches the detector will have traveled over a relatively shallow path through superficial scalp and bone tissues and is unlikely to have penetrated brain tissues underlying the bone of the neurocranium. Accordingly, when one of the shallow path emitters is activated, the adjacent detector produces a shallow path signal. The emitters **422** - **436** are spaced apart by about 30 mm center-to-center from the nearest detector and near infrared radiation emitted by these emitters thus travels over a deeper path through the underlying brain tissues before reaching the nearest detector. In one embodiment the penetration of the near infrared radiation from the emitters **422 - 436** is about 15 mm into the underlying brain tissues.

In one embodiment the shallow path signals are used as a basis for filtering noise from the signals produced between the emitters **422** - **436** and the respective nearest detectors. Noise may be induced by blood flow in the skin, blood flow in the neurocranium, the user's cardiac pulse, movement between the headset **108** and the users scalp, and ambient light, for example. The controller **110** may be operably configured to process the shallow path signals to determine shallow path noise and make corrections to the deep path signals when determining changes in blood oxygenation within the underlying brain tissues.

### Headset

The detector enclosures **112** and emitter enclosures **114** of the headset **108** act as a plurality of articulated segments which are urged toward the scalp of the user to cause contact between the associated light pipes **106** of the respective emitters **102** or detectors **104** and the scalp. Portions of the headset **108** that operate to urge the detector enclosures **112** toward the user's scalp are shown in exploded view in Figure **5****.** Referring to Figure **5**, the headset **108** includes a sprung band **500** that extends between the respective front portions **302** of adjacent detector enclosures **112.** The detector enclosures **112** are thus disposed spaced apart along the sprung band **500**, which has a curvature operable to conform to a corresponding lateral curvature of the user's scalp. The sprung band **500** urges the plurality of detectors **104** and the shallow path emitters **102** toward the user's scalp when the band is worn by the user, causing the associated light pipes **106** to contact the scalp.

The rear portions **200** of the emitter enclosures **114** are shown disposed in pairs, one of the pair being disposed forwardly with respect to the sprung band **500** and the other being disposed rearwardly with respect to the sprung band. Each of the rear portions **200** of the pair of emitter enclosures **114** has a spring **502** that joins between the rear portions and urges them toward the scalp when the headset **108** is worn by the user. The spring **502** causes the rear portions **200** and thus emitter enclosures **114** in each pair to be toed in to conform to a curvature of the user's neurocranium in a direction aligned with the sagittal plane.

As shown in Figure 2A and Figure **3**, the emitters **102** and detectors **104** are mounted on the circuit substrates **210** and **310.** Still referring to Figure **5**, in this embodiment the headset **108** also includes a detector flexible interconnect **504** and an emitter flexible interconnect **506**. The detector flexible interconnect **504** includes flexible tabs **508** that connect to each detector circuit substrate **310** within the detector enclosures **112**. Similarly, the emitter flexible interconnect **506** emitter flexible interconnect **506** includes flexible tabs **510** that connect to each emitter circuit substrate **210** within the emitter enclosures **114.** In one embodiment, the flexible interconnect and the plurality of circuit substrates may be formed as a unitary flexible circuit substrate.

### Electrical and control systems

A block diagram of the electrical and control components of the apparatus **100** is shown in Figure **6**. Referring to Figure **6** the headset controller is shown at **600** and includes a microcontroller **602.** In one embodiment the microcontroller **602** may be implemented using a ST Microelectronics controller such as the STM32F407VGT6 controller, which includes an on-board flash memory **610**, digital to analog converter (DAC) **612**, and a DAC buffer **614.** The memory **610** includes storage for instructions for directing the microcontroller to implement headset controller functionality and storage for other data generated. The DAC buffer **614** may be part of the memory **610** and is used for storing data defining a modulation waveform for driving the emitters **102.** The digital to analog converter **612** reads the data in the DAC buffer **614** and converts the digital waveform data into an emitter analog drive signal at an output **616.** The headset controller **600** further includes a drive signal conditioning block **604** that conditions and directs the analog drive signal to the various emitters **102** via the driver circuitry **212** and **312** shown in Figures 2A and **3.** The drive signal conditioning block **604** is controlled by a signal generated at an I/O output **620** of the microcontroller **602**, which facilitates selection of a particular emitter or emitters to be driven at any given time. A drive signal level provided to each individual emitter **102** is set by the microcontroller **602** via a scaling factor applied to the modulation waveform for driving the emitter.

The headset controller **600** also includes an analog to digital converter (ADC) **606.** Signals produced at each detector **104** are amplified and conditioned by the circuitry **312** shown in Figure **3** prior to conversion into digital data by the analog to digital converter **606.** In the embodiment shown, the output **616** of the digital to analog converter **612** is received by the analog to digital converter **606** at an input **626** for synchronous demodulation of the detector signals. The microcontroller **602** includes an input **618** for receiving data from the analog to digital converter **606.** In one embodiment the input **618** may be implemented as a Serial Peripheral Interface (SPI), which is capable of providing high bandwidth data transfer from the analog to digital converter **606.**

The headset controller **600** also includes a transmitter **608.** The transmitter **608** may be implemented as a Bluetooth wireless interface having a relatively low power consumption which permits the headset controller **600** to be run on battery power (not shown).

In this embodiment the headset controller **600** further includes a coupling sensor **622** in communication with an I/O input **624** of the microcontroller **602** for generating a coupling signal indicating a state of coupling between the plurality of light pipes of the emitters **102** and detectors **104** and the user's scalp. The coupling sensor **622** may be a capacitive sensor disposed on the headset **108** that produces a signal indicative of the proximity of the headset **108** to the scalp of the user. A reduction in sensed capacitance would be indicative of the headset **108** having been moved or removed such that the emitters **102** and detectors **104** are no longer in contact with the user's scalp. Alternatively or additionally, an acoustic sensor such as a microphone may be disposed on the headset **108** to generate a signal that in response to an ambient sound level at the microphone. An increase in sound level at the microphone may indicate that the headset **108** has been moved or removed. In other embodiments an accelerometer may be disposed on the headset **108** to provide inertial signals indicative of movement of the headset. Rapid movements of the headset **108** sensed by the accelerometer may be indicative that the headset **108** has been moved or removed. Another alternative would be to monitor ambient light signals experienced at one or more of the detectors **104.** When an ambient light component in the detector signal changes significantly, this may be indicative of the headset **108** being removed from the user's scalp. In one embodiment two or more of the alternative coupling sensors may be implemented to monitor the coupling conditions between the headset **108** and the user's scalp.

In this embodiment the headset controller **600** is in communication with the host controller **110**, which includes a microprocessor **630**, a memory **632**, a wireless radio **634**, and a display **636.** In the embodiment shown in Figure **1**, the host controller **110** is shown as a tablet computing device in which the display **636** is implemented as a touch sensitive screen for receiving user input. However in other embodiments the host controller **110** may be implemented using a conventional computing device such as a laptop or desktop computer or other computing device. The memory **632** includes storage **650** for storing codes that direct the microprocessor **630** to provide functions for implementing an operating system, such as an Android OS, iOS, Windows or other operating system. The memory **632** also includes storage **652** for storing codes that direct the microprocessor **630** to perform controller functions for interacting with and controlling the headset **108.** The memory **632** further incudes storage **654** storing data associated with performing brain activity monitoring.

In one embodiment the processor circuit **630** may include a graphic processing unit operably configured to accelerate processing of the signals from each of the plurality of detectors **104** to facilitate near real time presentation of results to the user.

The wireless radio **634** implements Bluetooth communication protocols for communicating with the transmitter **608** of the headset controller **600.** In other embodiments the wireless radio **634** may implement other wireless protocols for communicating with the transmitter **608**, which may be correspondingly configured to implement a wireless protocol other than the Bluetooth protocol.

### Headset controller process

Referring to Figure 7A, a flowchart depicting blocks of code for directing the headset controller **600** to perform a signal calibration is shown generally at **700.** The blocks generally represent codes that may be read from the memory **610** for directing the microcontroller **602** to perform signal acquisition. The actual code to implement each block may be written in any suitable program language, such as C, C++, C#, Java, and/or assembly code, for example.

The signal calibration process **700** begins at block **702** when user initiates a signal calibration at the host controller **110.** Block **704** directs the microcontroller **602** to determine whether a coupling criterion has been met by reading the coupling sensor **622** and comparing the coupling signal received at the I/O input **624** against a range of values determined to indicate that the coupling to the user's scalp is sufficient. If at block **704**, the coupling criterion is not met then the microcontroller **602** is directed to block **706**, which directs the microcontroller to transmit a notification to the host controller **110** for display to the user. The user may then relocate the headset on the scalp in an attempt to improve the coupling. Block **706** then directs the microprocessor **602** back to block **704** to re-check the coupling. When at block **704**, the coupling criterion is met, the microcontroller **602** is directed to block **708.** Block **708** directs the microcontroller **602** to acquire signals from the shallow path emitters **412** - **420** and the associated detectors. The signal acquisition process for emitter/detector pairs is shown in Figure 7B and is described in more detail later herein.

The signal calibration process **700** then continues at block **710**, which directs the microcontroller **602** to determine whether the signal level produced by each detector for each respective emitter/detector pair falls within a pre-determined signal level criterion. If the signal level criterion is not met at block **710**, the microcontroller **602** is directed to block **712**, which directs the microcontroller to determine whether the emitter signal level is at a maximum. If the signal level not yet maximized, block **712** directs the microcontroller **602** to block **714**, which directs the microcontroller to increase the emitter drive signal level for the emitter. The process then continues by repeating block **708** and **710**. If at block **712**, the signal level is already maximized, the microcontroller **602** is directed to block **716**, which directs the microcontroller to determine whether the detector gain is already at a maximum. If the detector gain has not already been maximized then block **716** directs the microcontroller **602** to block **718** and the gain of the detector is increased. Block **718** may also reduce the emitter drive signal level back to a lower level or a minimum level. Block **718** then directs the microcontroller **602** back to block **708** and blocks **708** and **710** are repeated with the increased detector gain.

If at block **716** the detector gain is at a maximum, the microcontroller **602** is directed to block **720.** Block **720** directs the microcontroller to transmit a notification message to the host controller **110** causing a message to be displayed for the user on the host controller. The user may adjust the headset position and elect to re-check, in which case block **722** directs the microcontroller **602** back to block **708** to repeat signal acquisition of the shallow path signals for the adjusted headset position. Alternatively, the user may elect to continue with the headset coupling as-is, in which case block **722** directs the microcontroller **602** to block **724.**

Block **724** then directs the microcontroller **602** to determine whether a cardiac pulse signal has been detected. The microcontroller **602** is directed to extract a cardiac pulse signal from the signal received at the detector, which is relatively strong compared to other signal components and also has a well-known waveform that facilitates extraction. If at block **724** the cardiac pulse signal is not detected, the microcontroller **602** is directed to block **726,** which directs the microcontroller to transmit a notification message to the host controller **110.** The user may then adjust the headset position and elect to re-check, in which case block **728** directs the microcontroller **602** back to block **708** to repeat the shallow path signal acquisition for the new headset position. Alternatively, the user may elect to continue with the coupling as-is, in which case block **728** directs the microcontroller **602** to block **730.**

If at block **724** the cardiac pulse signal is detected in the shallow path signal, the microcontroller **602** is directed to block **730.** The cardiac pulse signal provides an additional determination of the effectiveness of the coupling between the headset **108** and the user's scalp and is further used to perform filtering to remove physiological effects from signals not related to changes in blood oxygenation that are indicative of brain activity.

The signal calibration process **700** then continues at block **730**, which directs the microcontroller **602** to repeat the process for the deep path emitter/detector pairs substantially as described above in connection with the shallow path signals. The signal levels for driving the deep path emitters **422** - **436** and the detector gain is thus calibrated at blocks **730 - 740** to bring the signals within the signal level criterion for successful detection by the associated detectors. When the emitter drive signal level and detector gain are maximized and the user has adjusted the headset position and elected to re-check at block **744**, the microcontroller **602** is directed back to block **708** to repeat the signal acquisition for shallow path emitters at the new headset position.

The microcontroller **602** is also directed to determine whether the cardiac pulse signal is detected for the deep path emitter/detector pairs at block **746.** When the pulse signal is not detected at block **746** and the user has adjusted the headset position and elected to re-check at block **750**, the microcontroller **602** is directed back to block **708** to repeat the signal acquisition for shallow path emitters. If the signal level criterion is met at block **732** and the cardiac pulse is detected at block **746**, the signal calibration process **700** successfully ends at block **752.**

Referring to Figure 7B, a flowchart depicting blocks of code for directing the headset controller **600** to acquire signals is shown generally at **760.** The signal acquisition process **760** starts at block **762**, which directs the microcontroller **602** to commence data acquisition. In one embodiment the host controller **110** initiates the monitoring activity, but in other embodiments such as the signal calibration process **700** shown in Figure 7A the activity may be initiated at the headset **108.** Blocks **764** and **766** are optionally included to direct the microcontroller **602** to determine whether the coupling criterion has been met and to alert the user if not met. The host controller **110** may be operably configured to discontinue monitoring activities when the coupling criterion is indicative of a plurality of the emitters or detectors not being coupled to the scalp of the user.

If the coupling criterion is met at block **764**, block **768** then directs the microcontroller **602** to generate data representing a digital waveform for driving the emitters **102.** In one embodiment the modulation waveform is a sinewave having a frequency in the kilohertz range and a duration of about 4 milliseconds. Other embodiments may implement different waveforms, frequencies, and/or duration. Block **768** also directs the microcontroller **602** to store the waveform data in the DAC buffer **614.** In one embodiment the same digital waveform may be used for signal acquisition from each of the different emitter/detector pairs with a calibration scaling factor being applied to the waveform for each emitter/detector pair as determined by the signal calibration process **700**.

The signal acquisition process **760** then continues at block **770**, which directs the microcontroller **602** to select an emitter/detector pair that is to be activated for signal acquisition. The process **760** may be used to acquire signals from one emitter/detector pair or from a group of emitter/detector pairs, as in the case of the signal calibration process **700.** Each of the emitters **102** is paired with one of the detectors **104**, which as a pair define a measurement channel that can be activated. Block **770** directs the microcontroller **602** to cause the selected detector **104** to be configured for receiving signals via the analog to digital converter **606.** Block **770** also directs the microcontroller **602** to configure the drive signal conditioning block **604** via the I/O signal **620** to connect the selected emitter to produce near infrared radiation. In embodiments where the emitter is operably configured to produce near infrared radiation at multiple wavelengths, the drive signal conditioning block **604** may also configure the emitter to selectively enable each of the wavelength sources in the emitter to generate the respective wavelengths.

Block **772** then directs the microcontroller **602** to cause the digital to analog converter **612** to read the digital modulation waveform data stored in the DAC buffer **614** and to commence conversion of the digital data into an analog waveform. If the signal calibration process **700** has already been performed, the microcontroller **602** would also apply any determined calibration factor for driving the emitter at a signal level that produces sufficient signal at the associated detector. The analog waveform at the output **616** is thus connected through appropriate drive signal buffers in the drive signal conditioning block **604** to the selected emitter, which then generates a frequency burst having a duration and drive level set by the digital modulation data and the determined signal level calibration factor. The selected emitter couples near infrared radiation through the scalp, which travels through the underlying tissue such that at least a portion of reaches the selected detector and produces an analog signal representing the received near infrared radiation. In embodiments where the emitter **102** includes multiple wavelength sources, each wavelength is activated separately to facilitate generation of separate signals at the detector for each wavelength.

The signal acquisition process **760** then continues at block **774**, which directs the microcontroller **602** to cause the analog to digital converter **606** to convert the analog signal received at the selected detector into digital data representation, which is received at the input **618** of the microcontroller as a digital data stream. For an emitter **102** that operates at multiple wavelengths, digital data streams for each wavelength will thus be produced by the detector. Block **776** then directs the microcontroller **602** to process the digital data signals. During the signal calibration process **700** the microcontroller **602** processes the digital data representation to determine signal level and to extract a cardiac pulse signal, if present. Optionally, the processing at block **776** may further involve the microcontroller **602** causing the transmitter **608** to transmit the digital signal to the host controller **110** via the wireless Bluetooth connection for further processing by the host controller.

The process **760** then continues at block **778**, which directs the microcontroller **602** to determine whether signals have been acquired for all required emitter/detector pairs. If there remain further signals to be acquired, the microcontroller **602** is directed to block **770**, which directs the microcontroller to select the next emitter/detector pair for activation and blocks **772** - **778** are repeated for the next emitter/detector pair. If at block **778** there are no further signals to be acquired, the microcontroller **602** is directed to block **780** where the signal acquisition process ends.

Following completion of the signal calibration process **700**, a user assessment session may be commenced in which signals are acquired from the various the emitter/detector pairs for the duration of the session. For example, referring back to Figure **4**, the shallow path emitter **412** may be actuated together with the detector **400** to read the shallow path signal. This may be followed by the emitter **422** being actuated together with the detector **400** and then the emitter **430** together with the detector **400**. The activation sequence may then be repeated with the shallow path emitter **414** and detector **402**, followed by activation of the emitters **422** and then the emitter **430** together with the detector **400**. The activation sequence may then continue with emitters **424** and **432** and so on. In the emitter and detector layout shown in Figure **4**, the large arrows indicate **16** different deep path measurement channels that can be made by combining various ones of the emitters **102** with each detector **104.** Additional shallow path measurement channels also exist between each of the shallow path emitters **412** - **420** and the detectors **104.** These deep path measurement channels and shallow path measurement channels may be activated one-by-one in a sequence and various combinations of activation sequence may be implemented. In some embodiments two or more emitters and/or detectors may be simultaneously activated.

### Host controller process

Referring to Figure 8, a flowchart depicting blocks of code for directing the processor circuit of the host controller **110** to perform an assessment session is shown generally at **800.** The blocks generally represent codes that may be read from the controller application storage **652** in the memory **632** for directing the microprocessor **630** to perform the assessment. The actual code to implement each block may be written in any suitable program language, such as C, C++, C#, Java, and/or assembly code, for example.

A brain activity assessment commences at block **802** when a user launches the application and the microprocessor **630** is directed to execute the codes stored in the storage location **652** of the memory **632.** The application may initially go through a process of receiving user details that will be associated with the assessment session. Block **804** then directs the microprocessor **630** to attempt to establish a wireless connection between the wireless radio **634** of the host controller **110** and the transmitter **608** on the headset **108.** If at block **804** no wireless connection is established, the microprocessor **630** is directed to repeat block **804.**

If at block **804** a wireless connection with the headset **108** is established, the microprocessor **630** is directed to block **806.** Block **806** directs the microprocessor **630** to determine whether the coupling criterion for the headset **108** has been met. As described above in connection with the signal calibration process **700** and signal acquisition process **760,** when it is determined by the headset controller **600** that the headset **108** has been removed or moved on the user's scalp such that the coupling criterion is no longer met, a message is transmitted to the host controller **110.** Block **806** thus directs the microprocessor **630** to determine whether the coupling criterion is currently being met at the headset **108.** If the coupling criterion is not being met, block **808** directs the microprocessor **630** to cause a user notification (not shown) to be generated and displayed on the display **636** to prompt the user to put on or relocate the headset.

If at block **806** the coupling criterion is being met, block **806** directs the microprocessor **630** to block **810,** which directs the microprocessor to transmit an instruction via the wireless radio **634** to the headset controller **600** to initiate the signal calibration process **700** shown in Figure 7A. The assessment process **800** then continues at block **812,** which directs the microprocessor **630** to receive the digital signal representations generated by the detectors **104** on the headset **108** during the signal calibration process **700** and transmitted via the transmitter **608** to the host controller **110.** Block **812** also directs the microprocessor **630** to determine a signal level associated with each received signal.

Block **814** then directs the microprocessor **630** to generate data to cause a graphical depiction of the signal quality to be displayed on the display **636** of the host controller **110** to provide feedback to the user for properly locating the headset **108** on the user's scalp. Referring to Figure 9A, a first screenshot of the graphical depiction during signal quality evaluation is shown at **900** and includes a brain representation **902** including a plurality of dots **904** that act as a spatial representation of the deep path emitter/detector pairs along with display information indicating whether the signal intensity is within the detection range of the associated detector. An assessment region is depicted by coloring a subset **906** of the plurality of dots **904** (shown as **16** dark shaded regions in Figure 9A). Each dot **906** represents one of the signals generated by the headset **108** for the **16** different deep path signal acquisitions represented in Figure **4** by the large arrows. As shown in Figure **9**, some of the dots **906** have a smaller diameter than others. The diameter of each dot **906** is used as an indicator of signal level or quality for the associated path in accordance with a key **908** displayed alongside the brain representation **902.** The larger two dot sizes in the key **908** are shown to indicate "Acceptable" and "Excellent" signal quality, while the smaller dot sizes are associated with "poor" signal quality or "no signal".

Referring back to Figure **8**, the process **800** then continues at block **816**, which directs the microprocessor **630** to determine whether a signal level criterion is currently being met. If some of the signal levels are not at or above the "Acceptable" level, the signal level criterion is currently not being met and the microprocessor **630** is directed to block **814.** The headset controller **600** thus monitors the signal level produced at each detector and to control a level of near infrared radiation produced by the selectively actuated emitter and attempts to maintain the signal intensity within a detection range of the detector.

While at block **816** the signal level criterion is currently not being met, the graphical depiction **900** includes a status indicator **910** "Calibrating", which indicates that the signal quality is still being evaluated. If at block **816** the signal level criterion is currently being met, the microprocessor **630** is directed to block **818**, which directs the microprocessor **630** to change the displayed screen to the state shown in Figure 9B at **912**, where the status indicator **910** changes to "Continue" and all of the dots **906** in the brain representation **902** are shown in the "Acceptable" range or in the case of Figure 9B, in the "Excellent" range. The status indicator **910** thus prompts the user to continue with the assessment session.

The assessment process **800** then continues at block **820,** which directs the microprocessor **630** to wait until the user has activated the "Continue" status indicator **910** to continue with the assessment. When the user activates the "Continue" status indicator **910,** block **820** directs the microprocessor **630** to block **822.** Block **822** directs the microprocessor **630** to continue to receive the digital signal representations from the headset **108** and to process the signals to determine results for the assessment. Generally the signals received at the detectors will have significant noise and may also have significant components due to physiological processes such as the cardiac pulse that may obscure blood oxygenation information in the signals. The processing of the signals is described in more detail later herein.

Block **824** then directs the microprocessor **630** to generate and display results of the assessment. In one embodiment the microprocessor **630** causes a result screen shown in Figure 9 at **920** to be displayed while the assessment is in progress. The result screen **920** shows a brain representation **922.** Dots **926** associated with the various deep path signals are colored (shown as shaded in Figure 9C) to indicate a level of brain activity. The result screen **920** also includes a corresponding body representation **924,** in which regions of the body that are being activated are shaded to correspond to portions of the motor cortex of the brain that are associated with movement of those of the regions of the body. In this case the activity is a grasping action of the right hand. Various other portions of the body may be activated and the associated brain activity recorded while the results are displayed for the user as in the example shown in Figure 9C. The graphs **928** show changes in blood oxygenation over measured during an assessment. Each assessment may include series of trials of an exercise each being timed to have a period of rest followed by a period of activity (in this particular case **10** seconds of activity). The graphs **928** depict a user's brain activity during these exercises. The thin lines each represent multiple trials for an exercise over the **10** second activity window, with periods of rest outside the activity window. The thick line represents a mean of the multiple trials.

Referring back to Figure **8**, the assessment process **800** then continues at block **824**, which directs the microprocessor **630** to determine whether the user has discontinued the assessment session. If at block **824** the assessment session is not determined to have been discontinued, the microprocessor **630** is directed back to block **822** to continue receiving and processing signals. In one embodiment, block **824** further directs the microprocessor **630** to determine whether a message has been received at the host controller **110** indicating that the headset **108** has been removed or moved on the user's scalp such that the coupling criterion is no longer met. If at block **768** of the signal acquisition process **760,** a notification message is transmitted by the headset controller **600** that the coupling criteria is not being met, then the microprocessor **630** determines that the assessment should be discontinued.

If at block **824** the assessment session is determined to have been discontinued, the microprocessor **630** is directed to block **826**, where the microprocessor is directed to display a brain activity result summary. An example of the summary is shown in Figure 9D at **930** and includes assessment details for both right and left grasping activities along with other metrics for a selected measurement channel (in this case the R3D3 sensor indicated by the outlined circle in the summary **930.**

### Signal processing

As disclosed above, the processing of the signals at block **822** may involve steps that suppress components of the signal that do not relate to blood oxygenation changes within brain tissue. For example, physiological processes unrelated to brain activity such as the cardiac pulse, respiration, changes in blood pressure, have an effect on how the infrared radiation is absorbed by tissues through which the radiation travels between the emitters **102** and detectors **104.** Signals unrelated to brain activity are herein referred to as "contamination signals".

As disclosed above in connection with Figure **4**, the headset **108** implements multiple channels including deep path measurement channels where the emitter and detector are separated by a sufficient distance for infrared radiation to pass through brain tissues underlying the scalp before reaching the detector. The deep path measurement channels will thus generate deep path signals including signal components associated with blood oxygenation in the brain along with other components related to physiological processes (i.e. the contamination signals). The headset **108** also implements shallow path measurement channels where the emitter and detector are separated by a distance that is too short for the infrared radiation pass through brain tissue before reaching the detector. For the shallow path measurement channels the infrared radiation from the emitter does not reach the brain tissue and the resulting shallow path signals thus do not include components associated with blood oxygenation in the brain tissues. These shallow path signals thus provide a useful representation of the contamination signals and may be used to remove or filter contamination signal components from the deep path signals. The filtered deep path signals will have components related to blood oxygenation changes within brain tissue enhanced while contamination signal components are diminished.

There are some difficulties in performing the filtering of the deep path signals in that the physical processes and structures of the circulatory system may cause a variable delay between contamination signals generated for different deep path and shallow path measurement channels due to these channels being spaced apart about and within the user's neurocranium. There may also be differences between how the physiological process manifest for different deep path and shallow path measurement channels. A flowchart including blocks of code for directing the microprocessor **630** of the host controller **110** to implement block **822** of the process **800** is shown in Figure **10**. Referring to Figure **10**, the process **822** begins at block **1000**, which directs the microprocessor **630** to extract a cardiac pulse signal from the signal associated with each of the shallow path and deep path measurement channels. The cardiac pulse signal component is relatively strong compared to other signal components and also has a well-known waveform which facilitates extraction from the shallow path and deep path measurement channels. In other embodiments cyclic signals associated with other physiological processes such as respiration or blood pressure cyclic changes such as Mayer waves.

Block **1002** then directs the microprocessor **630** to determine the relative phase of each of the extracted cardiac pulse signals using one of the channels as a reference channel. Block **1004** then directs the microprocessor **630** to estimate a time delay for each channel relative to the reference channel (i.e. the reference channel is assumed to have zero time delay). The process **822** then continues at block **1006**, which directs the microprocessor **630** to align the deep path and shallow path signals for all the measurement channels. The processed signals at block **1006** thus have the variable delays due to the manifestation of the physiological processes on the respective signals all aligned so that the contamination signals are substantially aligned in time for all measurement channels.

In some embodiments, the absence of a cardiac pulse signal in detected signals may be taken as being indicative that the coupling criterion is no longer being met. The headset controller **600** or the host controller **110** may be operably configured to process the signal received by at least one of the detectors to extract a pulse signal representing a detected heartbeat of the user and to monitor the pulse signal to determine whether coupling between the emitters and detectors and the scalp of the user meets a coupling criterion. A dominant frequency may be extracted from the detected signals, and if the frequency falls within a frequency range based on the user's expected heartbeat frequency range, then the coupling criterion will be considered to be met. The cardiac pulse signal may thus be used in addition to or instead of the coupling signal produced by the coupling sensor **622** (shown in Figure **6**).

Block **1008** then directs the microprocessor **630** to process signals for each channel and at each wavelength to extract components associated with blood oxygenation. For example, in embodiments where a dual wavelength emitter having wavelengths of 750 nm and 850 nm is used, the components for each of these wavelengths may be extracted from the signals for each channel to yield a signal that is indicative of blood oxygenation associated with the channel.

Block **1010** then directs the microprocessor **630** to compute a first derivative of the signal produced at block **1008.** The inventors have found that signals that reflect a rate of change in blood oxygenation are less noisy than raw blood oxygenation signals.

The process **822** then continues at block **1012**, which directs the microprocessor **630** to preform principal component analysis (PCA) on the combined shallow path signals to generate an estimate for the contamination signals. In one embodiment the principal component analysis is applied more than once to the processed shallow path signals to differentiate between faster-cycling signals (for example cardiac pulse in the 0.5 Hz - 2 Hz range) and slower-cycling signals (for example respiration in the 0.01 Hz - 0.1 Hz range). For example, a first principal component analysis may be performed on the shallow path signals produced at block **1010.** This may be followed by a second principal component analysis on a high pass filtered version of the signals produced at block **1010** to selectively retain only fast-cycling signals. A further principal component analysis may be performed on a low pass filtered version of the signals produced at block **1010** to selectively retain only slow-cycling signals. The inventors have found that it is possible for a simple single-pass principal component analysis applied to the signals at block **1010** may capture either one of these fast or slow cycling components while possibly missing the other.

Block **1014** then directs the microprocessor **630** to compute a linear regression on the deep path signal produced at block **1010** for each deep path measurement channel. The linear regression predicts the deep path measurements as an additive function of all the contamination signals obtained by the principal component analysis, thereby estimating the influence of each contamination signal and providing a formula to then remove these influences from the deep path signals. The resulting signals have the influence of contamination signals substantially reduced to provide a signal representing changes in blood oxygenation that can be used to produce the result screen **920** shown in Figure 9C.

Functions described above as being implemented on either the host controller **110** or the headset controller **600** may be moved between the controllers or implemented on another controller (not shown). On other embodiments the headset controller **600** may be implemented using more than one microcontroller located on the headset **108**.

While specific embodiments have been described and illustrated, such embodiments should be considered illustrative only and not as limiting the disclosed embodiments as construed in accordance with the accompanying claims.

## Claims

1. An apparatus for monitoring brain activity of a user, the apparatus comprising:
a headset (108) comprising:
a sprung curved band (500) having a curvature operable to conform to a corresponding lateral curvature of the user's scalp over the user's crown and
a plurality of articulated segments (112, 114);
a plurality of spatially separated emitters (102) operable to generate infrared radiation disposed on the headset;
a plurality of spatially separated infrared radiation detectors (104) disposed on the headset;
wherein each one of the plurality of emitters (102) and detectors (104) has an associated light pipe (106) operable to couple infrared radiation from the emitter into the scalp or to couple infrared radiation from the scalp to the detector;
a plurality of light pipes (106), wherein each light pipe (106) comprises:
a coupling surface for coupling infrared radiation between the light pipe and the emitter or detector;
a distal lens (220) operably configured to contact the scalp and direct infrared radiation to or from the light pipe;
a guide portion extending between the coupling surface and the distal lens;
wherein a length of at least about 7 mm of each of the plurality of light pipes protrudes outwardly from a surface of the headset;
wherein each light pipe of said plurality of light pipes comprises a low durometer material that is optically transmissive at wavelengths associated with the infrared radiation, the low durometer material facilitating comfortable optical contact with the scalp of the user; wherein the light pipe material has a durometer in a range of between about Shore A durometer 30 and about Shore A durometer 90,
a sheath (222) surrounding at least a portion of the guide portion (218) of the light pipe, wherein said sheath is optically absorbent at the wavelengths emitted by the emitters (102); and wherein an outer surface of the sheath (222) is configured to divert the user's hair away from the distal lens (220) when the light pipe (106) is in contact with the scalp, and
a transmitter (608);
wherein the plurality of spatially separated emitters comprise:
at least one emitter (102) disposed proximate to one of the plurality of detectors (104) and wherein the detector is operable to produce a shallow path signal representing an intensity of infrared radiation generated after traveling along a path through scalp and bone tissue between the at least one emitter and the detector; and
at least one emitter (102) disposed spaced apart from one or more of the plurality of detectors (104) and wherein the one or more detectors are operable to produce a deep path signal representing an intensity of infrared radiation generated after traveling along a path through the underlying brain tissue between the at least one emitter and the one or more detectors,
wherein each of the plurality of spatially separated emitters (102) comprises a light emitting diode operably configured to produce the infrared radiation at a plurality of wavelengths selected to cause the detector to produce signals that facilitate determination of a blood oxygenation state of the brain tissue underlying each of the spatially separated emitters and associated detectors, the blood oxygenation state being indicative of local cerebral hemodynamics within the brain tissue and facilitating a determination of neural activity within the user's brain,
wherein the plurality of articulated segments disposed forwardly or rearwardly with respect to the sprung band;
wherein each articulated segment (112, 114) supports at least one emitter (102) of said plurality of spatially separated emitters or at least one detector (104) of said plurality of spatially separated detectors, the articulated segments each being urged by the sprung band (500) toward the scalp of the user to cause contact between the associated light pipes of the respective emitters or detectors and the scalp;
wherein each detector (104) is operable to produce a signal representing an intensity of infrared radiation generated by a selectively actuated one of the plurality of emitters (102) and received at the detector after traveling on a path through underlying brain tissue, and
wherein said transmitter (608) is operable to wirelessly transmit the signals to a controller (110) remotely disposed with respect to the headset for processing, wherein said controller (110) is operably configured to:
process the signals from each detector to determine changes in blood oxygenation within the brain tissue along the path between the respective emitter and detector; and
generate a spatial representation of brain activity within in the user's brain based on the processed signals;
the apparatus further comprising at least one coupling sensor (622) operably configured to generate a coupling signal indicating a state of coupling between the plurality of light pipes and the user's scalp, and wherein the controller is operably configured to discontinue the monitoring in response to the coupling signal indicating that a coupling criterion is not being met.

2. The apparatus of claim 1, further comprising a headset controller (600) disposed on the headset and operably configured to control functions of the transmitter, the emitters, and the detectors.

3. The apparatus of claim 1, wherein the infrared radiation comprises near infrared radiation.

4. The apparatus of claim 1, wherein the plurality of wavelengths comprise at least first and second wavelengths selected to fall on either side of the isobestic point for oxygenation and deoxygenation of blood hemoglobin; or
wherein the light emitting diode associated with each of the plurality of emitters is mounted within a headset, the headset being operable to support the plurality of emitters and plurality of detectors in contact with the user's scalp when worn by the user.

5. The apparatus of claim 1, wherein the controller (110) is operably configured: to activate selected emitters and detectors to generate signals associated with different paths of travel of the infrared radiation through the brain tissue; or to process the shallow path signals to determine shallow path noise, the shallow path noise being used as a basis for filtering the deep path signal to determine the changes in blood oxygenation within the brain tissue.

6. The apparatus of claim 5, wherein the controller is operably configured to process the signals by:
aligning a phase of each of the shallow path signals and deep path signals based on a physiological process component in the signals;
performing a principle component analysis on the shallow path signals to determine contamination components associated with physiological processes other than changes in blood oxygenation within the brain tissue; and
removing the contamination components from the deep path signals to provide signals representing changes in blood oxygenation within the brain tissue from which the effects of other physiological processes have been filtered.

7. The apparatus of claim 6, wherein performing the principle component analysis comprises:
filtering the shallow path signals to separate the shallow path signals into slow-cycling signals associated with slow-cycling physiological processes and fast-cycling signals associated with fast-cycling physiological processes; and
performing principle component analysis on each of the shallow path signals, the slow-cycling signals and the fast-cycling signals; or wherein the controller is operably configured to, prior to performing the principle component analysis:
process the phase aligned shallow path signals to generate signals representing oxygenation and deoxygenation of blood hemoglobin; and
take a first derivative of the signals representing oxygenation and deoxygenation of blood hemoglobin.

8. The apparatus of claim 1, wherein:
(a) the length of each light pipe (106) is between about 7 millimeters and 15 millimeters; and wherein a length of at least about 7 mm of the light pipe protrudes outwardly from a surface of the headset; or
(b) wherein the guide portion of the light pipe has a generally cylindrical shape and has a diameter selected to cause total internal reflection of infrared radiation incident at inner surfaces of the guide portion.

9. The apparatus of claim 8, wherein a cross sectional area of the guide portion is smaller than a cross sectional area of the coupling surface and the light pipe further comprises a tapered transition between the coupling surface and the guide portion and wherein a taper angle of the tapered transition is selected to prevent infrared radiation leakage from the tapered transition, the tapered transition further providing for mounting of the light pipe to the emitter or detector.

10. The apparatus of claim 1, wherein the coupling surface of the light pipe is operably configured to directly contact a radiating surface of the emitter or a radiation receiving surface of the detector for coupling infrared radiation between the light pipe and the detector.

11. The apparatus of claim 1, wherein each of the plurality of articulated segments (112, 114) is operably configured to mount a circuit substrate and wherein at least one detector or emitter is mounted on each circuit substrate; further comprising a flexible interconnect (504) interconnecting between a headset controller (600) and the plurality of circuit substrates (210); and wherein the flexible interconnect and the plurality of circuit substrates are formed as a unitary flexible circuit substrate.

12. The apparatus of claim 1, wherein the controller is operably configured to monitor the signal level produced at each detector and to control a level of infrared radiation produced by the selectively actuated emitter to maintain the intensity within a detection range of the detector.

13. The apparatus of claim 12, wherein the controller is further operably configured to generate display data for display as a graphic user interface, GUI, on a screen in communication with the controller, the GUI including a spatial representation of at least one of the emitters and detectors along with display information indicating whether the signal intensity is within the detection range of the associated detector; or wherein the controller is operably configured to discontinue the monitoring when the signals received from the detectors no longer meet a coupling criterion indicative of a plurality of the emitters or detectors being coupled to the scalp of the user.

14. The apparatus of claim 1, wherein the at least one coupling sensor comprises at least one of:
a capacitive sensor that produces a signal indicative of a proximity of the apparatus to the scalp;
an acoustic sensor that produces a signal in response to an ambient sound level;
an inertial sensor that produces a signal indicative of movement of the apparatus; or
one or more of the detectors, wherein an ambient light component in the signal produced by the one or more detectors is indicative of the apparatus being removed from the scalp and the detector being subject to ambient light radiation.

15. The apparatus of claim 13, wherein the controller is operably configured to process the signal received by at least one of the detectors to extract a cardiac pulse signal representing a detected heartbeat of the user and to monitor the pulse signal to determine whether coupling between the emitters and detectors and the scalp of the user meets a coupling criterion.

16. The apparatus of claim 15, wherein the controller is operably configured: to process the signals by extracting a dominant frequency from the signals that falls within a frequency range based on the user's expected heartbeat frequency range; and/or to discontinue the monitoring when the cardiac pulse signals received from the detectors no longer meet the coupling criterion.

17. The apparatus of claim 1, wherein the controller is operably configured to monitor time variations in changes in blood oxygenation within the brain tissue in a region underlying each detector and selectively actuated emitter and to generate data metrics representing a degree of brain activation in each region; or
wherein the controller is further operably configured to generate display data for display as a graphic user interface, GUI, on a screen in communication with the controller, the GUI including a representation of regions of the user's body that correspond to regions of the user's brain that are indicated by the changes in blood oxygenation within the brain tissue to be actuated; or
wherein the controller comprises a processor circuit, the processor circuit including a graphic processing unit operably configured to accelerate processing of the signals from each of the plurality of detectors to facilitate near real time presentation of results to the user.

## Patentansprüche

1. Vorrichtung zum Überwachen der Gehirnaktivität eines Benutzers, wobei die Vorrichtung umfasst:
einen Kopfhörer (108), umfassend
ein gefedertes gekrümmtes Band (500) mit einer Krümmung, das so betrieben werden kann, dass es sich an eine entsprechende seitliche Krümmung der Kopfhaut des Benutzers über dem Scheitel des Benutzers anpasst; und
eine Vielzahl von gelenkigen Segmenten (112, 114);
eine Vielzahl von räumlich getrennten Emittern (102), die so betrieben werden können, dass sie Infrarotstrahlung erzeugen, und an dem Kopfhörer angeordnet sind;
eine Vielzahl von räumlich getrennten Infrarotstrahlungsdetektoren (104), die an dem Kopfhörer angeordnet sind;
wobei jeder der Vielzahl von Emittern (102) und Detektoren (104) einen zugehörigen Lichtleiter (106) aufweist, der so betrieben werden kann, dass er Infrarotstrahlung von dem Emitter in die Kopfhaut koppelt oder Infrarotstrahlung von der Kopfhaut zu dem Detektor koppelt;
eine Vielzahl von Lichtleitern (106), wobei jeder Lichtleiter (106) umfasst:
eine Kopplungsfläche zum Koppeln von Infrarotstrahlung zwischen dem Lichtleiter und dem Emitter oder Detektor;
eine distale Linse (220), die operativ konfiguriert ist, um die Kopfhaut zu berühren und Infrarotstrahlung zu oder von dem Lichtleiter zu leiten;
einen Führungsabschnitt, der sich zwischen der Kopplungsfläche und der distalen Linse erstreckt;
wobei eine Länge von mindestens etwa 7 mm von jedem der Vielzahl von Lichtleitern nach außen aus einer Oberfläche des Kopfhörers herausragt;
wobei jeder Lichtleiter der Vielzahl von Lichtleitern ein Material mit niedriger Härte umfasst, das bei Wellenlängen, die mit der Infrarotstrahlung einhergehen, optisch durchlässig ist, wobei das Material mit niedriger Härte einen komfortablen optischen Kontakt mit der Kopfhaut des Benutzers ermöglicht; wobei das Lichtleitermaterial einen Härtegrad in einem Bereich zwischen etwa Shore A Durometer 30 und etwa Shore A Durometer 90 aufweist,
eine Umhüllung (222), die mindestens einen Teil des Führungsabschnitts (218) des Lichtleiters umgibt, wobei die Umhüllung bei den von den Emittern (102) emittierten Wellenlängen optisch absorbierend ist; und wobei eine Außenfläche der Umhüllung (222) so konfiguriert ist, dass sie das Haar des Benutzers von der distalen Linse (220) wegleitet, wenn der Lichtleiter (106) in Kontakt mit der Kopfhaut ist, und
einen Transmitter (608);
wobei die Vielzahl von räumlich getrennten Emittern umfasst:
mindestens einen Emitter (102), der in der Nähe eines der Vielzahl von Detektoren (104) angeordnet ist, und wobei der Detektor betreibbar ist, um ein Signal auf einem oberflächennahen Pfad zu erzeugen, das eine Intensität von Infrarotstrahlung darstellt, die nach dem Durchlaufen eines Pfades durch Kopfhaut und Knochengewebe zwischen dem mindestens einen Emitter und dem Detektor erzeugt wird; und
mindestens einen Emitter (102), der von einem oder mehreren der Vielzahl von Detektoren (104) beabstandet angeordnet ist, und wobei der eine oder die mehreren Detektoren betreibbar sind, um ein Signal auf einem tiefen Pfad zu erzeugen, das eine Intensität von Infrarotstrahlung darstellt, die nach dem Durchlaufen eines Pfades durch das darunter liegende Hirngewebe zwischen dem mindestens einen Emitter und dem einen oder den mehreren Detektoren erzeugt wird,
wobei jeder der Vielzahl von räumlich getrennten Emittern (102) eine lichtemittierende Diode umfasst, die operativ konfiguriert ist, um die Infrarotstrahlung bei einer Vielzahl von Wellenlängen zu erzeugen, die ausgewählt sind, um den Detektor zu veranlassen, Signale zu erzeugen, die die Bestimmung eines Blutsauerstoffversorgungszustands des Hirngewebes, das jedem der räumlich getrennten Emitter und zugehörigen Detektoren unterliegt, erleichtern, wobei der Zustand der Blutsauerstoffversorgung ein Indikator für die lokale zerebrale Hämodynamik innerhalb des Hirngewebes ist und eine Bestimmung der neuralen Aktivität innerhalb des Gehirns des Benutzers erleichtert,
wobei die Vielzahl der gelenkigen Segmente in Bezug auf das gefederte Band nach vorne oder nach hinten angeordnet ist;
wobei jedes gelenkige Segment (112, 114) mindestens einen Emitter (102) der Vielzahl von räumlich getrennten Emittern oder mindestens einen Detektor (104) der Vielzahl von räumlich getrennten Detektoren stützt, wobei die gelenkigen Segmente jeweils durch das gefederte Band (500) in Richtung der Kopfhaut des Benutzers gedrängt werden, um einen Kontakt zwischen den zugehörigen Lichtleitern der jeweiligen Emitter oder Detektoren und der Kopfhaut zu bewirken;
wobei jeder Detektor (104) betreibbar ist, um ein Signal zu erzeugen, das eine Intensität von Infrarotstrahlung darstellt, die von einem selektiv betätigten der Vielzahl von Emittern (102) erzeugt und am Detektor empfangen wird, nachdem sie einen Pfad durch darunterliegendes Hirngewebe zurückgelegt hat, und
wobei der Transmitter (608) so betreibbar ist, dass er die Signale drahtlos an eine Steuereinheit (110) überträgt, die von dem Kopfhörer entfernt zur Verarbeitung angeordnet ist, wobei die Steuereinheit (110) operativ so konfiguriert ist, dass sie:
die Signale von jedem Detektor verarbeitet, um Änderungen der Blutsauerstoffversorgung innerhalb des Hirngewebes entlang des Pfades zwischen dem jeweiligen Emitter und dem Detektor zu bestimmen; und
eine räumliche Darstellung der Gehirnaktivität im Gehirn des Benutzers auf der Grundlage der verarbeiteten Signale erzeugt;
wobei die Vorrichtung ferner mindestens einen Kopplungssensor (622) umfasst, der operativ so konfiguriert ist, dass er ein Kopplungssignal erzeugt, das einen Zustand der Kopplung zwischen der Mehrzahl von Lichtleitern und der Kopfhaut des Benutzers anzeigt, und wobei die Steuerung operativ so konfiguriert ist, dass sie die Überwachung in Reaktion auf das Kopplungssignal, das anzeigt, dass ein Kopplungskriterium nicht erfüllt ist, unterbricht.

2. Vorrichtung nach Anspruch 1, die ferner eine Kopfhörer-Steuereinheit (600) umfasst, die an dem Kopfhörer angeordnet und operativ so konfiguriert ist, dass sie die Funktionen des Transmitters, der Emitter und der Detektoren steuert.

3. Vorrichtung nach Anspruch 1, wobei die Infrarotstrahlung Nahinfrarotstrahlung umfasst.

4. Vorrichtung nach Anspruch 1, wobei die Vielzahl von Wellenlängen mindestens eine erste und eine zweite Wellenlänge umfasst, die so ausgewählt sind, dass sie auf beide Seiten des isobestischen Punkts für Oxygenierung und Deoxygenierung von Bluthämoglobin fallen; oder wobei die lichtemittierende Diode, die mit jedem der Vielzahl von Emittern verbunden ist, innerhalb eines Kopfhörers angebracht ist, wobei der Kopfhörer so betreibbar ist, dass er die Vielzahl von Emittern und die Vielzahl von Detektoren in Kontakt mit der Kopfhaut des Benutzers hält, wenn er von dem Benutzer getragen wird.

5. Vorrichtung nach Anspruch 1, wobei die Steuereinheit (110) operativ konfiguriert ist:
um ausgewählte Emitter und Detektoren zu aktivieren, um Signale zu erzeugen, die mit verschiedenen Propagationspfaden der Infrarotstrahlung durch das Hirngewebe verbunden sind; oder die Signale auf dem oberflächennahen Pfad zu verarbeiten, um das Rauschen auf dem oberflächennahen Pfad zu bestimmen, wobei das Rauschen auf dem oberflächennahen Pfad als Grundlage für die Filterung des Signals auf dem tiefen Pfad verwendet wird, um die Änderungen der Blutsauerstoffversorgung innerhalb des Hirngewebes zu bestimmen.

6. Vorrichtung nach Anspruch 5, wobei die Steuereinheit operativ konfiguriert ist, um die Signale zu verarbeiten durch:
Ausrichten einer Phase jedes der Signale auf dem oberflächennahen Pfad und der Signale auf dem tiefen Pfad basierend auf einer physiologischen Verarbeitungskomponente in den Signalen;
Durchführen einer Hauptkomponentenanalyse der Signale auf dem oberflächennahen Pfad, um Verunreinigungskomponenten zu bestimmen, die mit anderen physiologischen Prozessen als den Änderungen in dem Blutsauerstoffgehalt innerhalb des Hirngewebes zusammenhängen; und
Entfernen der Verunreinigungskomponenten aus den Signalen auf dem tiefen Pfad, um Signale bereitzustellen, die Veränderungen der Blutsauerstoffversorgung innerhalb des Hirngewebes darstellen, aus denen die Auswirkungen anderer physiologischer Prozesse herausgefiltert wurden.

7. Vorrichtung nach Anspruch 6, wobei die Durchführung der Hauptkomponentenanalyse umfasst:
Filtern der Signale auf dem oberflächennahen Pfad, um die Signale auf dem oberflächennahen Pfad in langsam zyklierende Signale, die mit langsam zyklierenden physiologischen Prozessen verbunden sind, und schnell zyklierende Signale, die mit schnell zyklierenden physiologischen Prozessen verbunden sind, zu trennen; und
Durchführen einer Hauptkomponentenanalyse an jedem der Signale auf dem oberflächennahen Pfad, den langsam zyklierenden Signalen und den schnell zyklierenden Signalen; oder wobei die Steuereinheit operativ konfiguriert ist, um vor der Durchführung der Hauptkomponentenanalyse:
die phasenausgerichteten Signale auf dem oberflächennahen Pfad zu verarbeiten, um Signale zu erzeugen, die die Oxygenierung und Deoxygenierung von Bluthämoglobin darstellen; und
eine erste Ableitung der Signale zu nehmen, die die Oxygenierung und Deoxygenierung von Bluthämoglobin darstellen.

8. Die Vorrichtung nach Anspruch 1, wobei:
(a) die Länge jedes Lichtleiters (106) zwischen etwa 7 Millimetern und 15 Millimetern liegt; und wobei eine Länge von mindestens etwa 7 mm des Lichtleiters von einer Oberfläche des Kopfhörers nach außen ragt; oder
(b) wobei der Führungsabschnitt des Lichtleiters eine allgemein zylindrische Form hat und einen Durchmesser aufweist, der so gewählt ist, dass er eine innere Totalreflexion von Infrarotstrahlung verursacht, die auf Innenflächen des Führungsabschnitts einfällt.

9. Vorrichtung nach Anspruch 8, wobei eine Querschnittsfläche des Führungsabschnitts kleiner ist als eine Querschnittsfläche der Kopplungsfläche und der Lichtleiter ferner einen verjüngten Übergang zwischen der Kopplungsfläche und dem Führungsabschnitt aufweist und
wobei ein Verjüngungswinkel des verjüngten Übergangs so gewählt ist, dass ein Austreten von Infrarotstrahlung aus dem verjüngten Übergang verhindert wird, wobei der verjüngte Übergang ferner für die Befestigung des Lichtleiters an dem Emitter oder Detektor sorgt.

10. Vorrichtung nach Anspruch 1, wobei die Kopplungsfläche des Lichtleiters operativ so konfiguriert ist, dass sie direkt mit einer strahlenden Oberfläche des Emitters oder einer Strahlung empfangenden Oberfläche des Detektors zur Kopplung von Infrarotstrahlung zwischen dem Lichtleiter und dem Detektor in Kontakt steht.

11. Vorrichtung nach Anspruch 1, wobei jedes der Vielzahl von gelenkigen Segmenten (112, 114) operativ konfiguriert ist, um ein Schaltungssubstrat anzubringen, und wobei mindestens ein Detektor oder Emitter auf jedem Schaltungssubstrat angebracht ist; ferner umfassend eine flexible Zwischenverbindung (504), die eine Verbindung zwischen einer Kopfhörer-Steuereinheit (600) und der Vielzahl von Schaltungssubstraten (210) herstellt;
und wobei die flexible Zwischenverbindung und die Vielzahl von Schaltungssubstraten als ein einheitliches flexibles Schaltungssubstrat ausgebildet sind.

12. Vorrichtung nach Anspruch 1, wobei die Steuereinheit operativ so konfiguriert ist, dass sie den an jedem Detektor erzeugten Signalpegel überwacht und den Pegel der Infrarotstrahlung steuert, die von dem selektiv betätigten Emitter erzeugt wird, um die Intensität innerhalb eines Detektionsbereichs des Detektors zu halten.

13. Vorrichtung nach Anspruch 12, wobei die Steuereinheit ferner operativ konfiguriert ist, um Anzeigedaten zur Anzeige als grafische Benutzeroberfläche (GUI) auf einem Bildschirm in Kommunikation mit der Steuereinheit zu erzeugen, wobei die GUI eine räumliche Darstellung von mindestens einem der Emitter und Detektoren zusammen mit Anzeigeinformationen beinhaltet, die anzeigen, ob die Signalintensität in dem Detektionsbereich des zugeordneten Detektors liegt,
oder wobei die Steuereinheit operativ konfiguriert ist, um die Überwachung zu unterbrechen, wenn die von den Detektoren empfangenen Signale ein Kopplungskriterium nicht mehr erfüllen, das anzeigt, dass eine Vielzahl der Emitter oder Detektoren mit der Kopfhaut des Benutzers gekoppelt sind.

14. Die Vorrichtung nach Anspruch 1, wobei der mindestens eine Kopplungssensor mindestens eines der folgenden Elemente umfasst:
einen kapazitiven Sensor, der ein Signal erzeugt, das die Nähe des Geräts zur Kopfhaut anzeigt;
einen akustischen Sensor, der ein Signal in Reaktion auf einen Umgebungsschallpegel erzeugt;
einen Trägheitssensor, der ein Signal erzeugt, das eine Bewegung des Geräts anzeigt; oder
einen oder mehrere der Detektoren, wobei eine Umgebungslichtkomponente in dem von dem einen oder den mehreren Detektoren erzeugten Signal anzeigt, dass die Vorrichtung von der Kopfhaut entfernt ist und der Detektor Umgebungslichtstrahlung ausgesetzt ist.

15. Vorrichtung nach Anspruch 13, wobei die Steuereinheit operativ konfiguriert ist, um das von mindestens einem der Detektoren empfangene Signal zu verarbeiten, um ein Herzpulssignal zu extrahieren, das einen detektierten Herzschlag des Benutzers repräsentiert, und das Pulssignal zu überwachen, um zu bestimmen, ob die Kopplung zwischen den Emittern und Detektoren und der Kopfhaut des Benutzers ein Kopplungskriterium erfüllt.

16. Vorrichtung nach Anspruch 15, wobei die Steuereinheit operativ konfiguriert ist, um die Signale zu verarbeiten, indem eine dominante Frequenz aus den Signalen extrahiert wird, die in einen Frequenzbereich fällt, der auf dem erwarteten Herzschlagfrequenzbereich des Benutzers basiert; und/oder die Überwachung zu unterbrechen, wenn die von den Detektoren empfangenen Herzpulssignale das Kopplungskriterium nicht mehr erfüllen.

17. Vorrichtung nach Anspruch 1, wobei die Steuereinheit operativ konfiguriert ist, um zeitliche Schwankungen der Änderungen der Blutsauerstoffversorgung im Hirngewebe in einem Bereich, der jedem Detektor und selektiv betätigten Emitter unterliegt, zu überwachen und Datenmetriken zu erzeugen, die einen Grad der Hirnaktivierung in jedem Bereich darstellen; oder
wobei die Steuereinheit ferner operativ konfiguriert ist, um Anzeigedaten zur Anzeige als grafische Benutzerschnittstelle, GUI, auf einem Bildschirm in Kommunikation mit der Steuereinheit zu erzeugen, wobei die GUI eine Darstellung von Bereichen des Körpers des Benutzers enthält, die Bereichen des Gehirns des Benutzers entsprechen, die durch die Änderungen der Blutsauerstoffversorgung innerhalb des zu aktivierenden Gehirngewebes angezeigt werden; oder
wobei die Steuereinheit eine Prozessorschaltung umfasst, wobei die Prozessorschaltung eine Grafikverarbeitungseinheit enthält, die operativ konfiguriert ist, um die Verarbeitung der Signale von jedem der Vielzahl von Detektoren zu beschleunigen, um die Darstellung der Ergebnisse für den Benutzer nahezu in Echtzeit zu erleichtern.

## Revendications

1. Appareil pour la surveillance de l'activité cérébrale d'un utilisateur, comprenant :
un casque (108) comprenant :
une bande incurvée élastique (500) ayant une courbure servant à se conformer à une courbure latérale correspondante du cuir chevelu de l'utilisateur sur la couronne de l'utilisateur et
une pluralité de segments articulés (112, 114) ;
une pluralité d'émetteurs (102) séparés dans l'espace servant à générer un rayonnement infrarouge, disposés sur le casque ;
une pluralité de détecteurs de rayonnement infrarouge (104) séparés dans l'espace, disposés sur le casque ;
dans lequel chacun de la pluralité d'émetteurs (102) et de détecteurs (104) possède un conduit de lumière (106) associé servant à coupler un rayonnement infrarouge provenant de l'émetteur dans le cuir chevelu ou à coupler un rayonnement infrarouge provenant du cuir chevelu au détecteur ;
une pluralité de conduits de lumière (106), dans lequel chaque conduit de lumière (106) comprend :
une surface de couplage pour le couplage d'un rayonnement infrarouge entre le conduit de lumière et l'émetteur ou le détecteur ;
une lentille distale (220) configurée de manière opérationnelle pour entrer en contact avec le cuir chevelu et diriger un rayonnement infrarouge vers le conduit de lumière ou provenant de celui-ci ;
une partie guide s'étendant entre la surface de couplage et la lentille distale ;
dans lequel une longueur d'au moins environ 7 mm de chacun de la pluralité de conduits de lumière fait saillie vers l'extérieur à partir d'une surface du casque ;
dans lequel chaque conduit de lumière de ladite pluralité de conduits de lumière comprend un matériau à faible dureté qui est optiquement transmissif à des longueurs d'onde associées au rayonnement infrarouge, le matériau à faible dureté facilitant un contact optique confortable avec le cuir chevelu de l'utilisateur ; dans lequel le matériau de conduit de lumière possède une dureté dans une plage d'entre environ 30 de dureté Shore A et environ 90 de dureté Shore A,
une gaine (222) encerclant au moins une partie de la partie guide (218) du conduit de lumière, dans lequel ladite gaine est optiquement absorbante aux longueurs d'onde émises par les émetteurs (102) ; et dans lequel une surface externe de la gaine (222) est configurée pour éloigner les cheveux de l'utilisateur de la lentille distale (220) lorsque le conduit de lumière (106) est en contact avec le cuir chevelu, et
un transmetteur (608) ;
dans lequel la pluralité d'émetteurs séparés dans l'espace comprennent :
au moins un émetteur (102) disposé à proximité de l'un de la pluralité de détecteurs (104) et dans lequel le détecteur sert à produire un signal de passage superficiel représentant une intensité d'un rayonnement infrarouge généré après un déplacement le long d'un passage à travers le cuir chevelu et du tissu osseux entre l'au moins un émetteur et le détecteur ; et
au moins un émetteur (102) disposé espacé de l'un ou de plusieurs détecteurs parmi la pluralité de détecteurs (104) et dans lequel les un ou plusieurs détecteurs servent à produire un signal de passage en profondeur représentant une intensité d'un rayonnement infrarouge généré après un déplacement le long d'un passage à travers le tissu cérébral sous-jacent entre l'au moins un émetteur et les un ou plusieurs détecteurs,
dans lequel chacun de la pluralité d'émetteurs (102) séparés dans l'espace comprend une diode électroluminescente configurée de manière opérationnelle pour produire le rayonnement infrarouge à une pluralité de longueurs d'onde sélectionnées pour amener le détecteur à produire des signaux qui facilitent la détermination d'un état d'oxygénation sanguine du tissu cérébral sous-jacent à chacun des émetteurs séparés dans l'espace et des détecteurs associés, l'état d'oxygénation sanguine étant indicatif de l'hémodynamique cérébrale locale au sein du tissu cérébral et facilitant une détermination d'activité neuronale au sein du cerveau de l'utilisateur,
dans lequel la pluralité de segments articulés sont disposés vers l'avant ou vers l'arrière par rapport à la bande élastique ;
dans lequel chaque segment articulé (112, 114) porte au moins un émetteur (102) de ladite pluralité d'émetteurs séparés dans l'espace ou au moins un détecteur (104) de ladite pluralité de détecteurs séparés dans l'espace, les segments articulés étant chacun sollicités par la bande élastique (500) vers le cuir chevelu de l'utilisateur pour provoquer un contact entre les conduits de lumière associés des émetteurs ou détecteurs respectifs et le cuir chevelu ;
dans lequel chaque détecteur (104) sert à produire un signal représentant une intensité d'un rayonnement infrarouge généré par un émetteur sélectivement actionné parmi la pluralité d'émetteurs (102) et reçu au niveau du détecteur après un déplacement sur un passage à travers du tissu cérébral sous-jacent, et
dans lequel ledit transmetteur (608) sert à transmettre sans fil les signaux à un dispositif de commande (110) disposé à distance par rapport au casque pour un traitement, dans lequel ledit dispositif de commande (110) est configuré de manière opérationnelle pour :
traiter les signaux provenant de chaque détecteur pour déterminer des changements de l'oxygénation sanguine au sein du tissu cérébral le long du passage entre l'émetteur et le détecteur respectifs ; et
générer une représentation spatiale de l'activité cérébrale au sein du cerveau de l'utilisateur sur la base des signaux traités ;
l'appareil comprenant en outre au moins un capteur de couplage (622) configuré de manière opérationnelle pour générer un signal de couplage indiquant un état de couplage entre la pluralité de conduits de lumière et le cuir chevelu de l'utilisateur, et dans lequel le dispositif de commande est configuré de manière opérationnelle pour interrompre la surveillance en réponse au signal de couplage indiquant qu'un critère de couplage n'est pas respecté.

2. Appareil selon la revendication 1, comprenant en outre un dispositif de commande de casque (600) disposé sur le casque et configuré de manière opérationnelle pour commander des fonctions du transmetteur, des émetteurs et des détecteurs.

3. Appareil selon la revendication 1, dans lequel le rayonnement infrarouge comprend un rayonnement infrarouge proche.

4. Appareil selon la revendication 1, dans lequel la pluralité de longueurs d'onde comprennent au moins des première et seconde longueurs d'onde sélectionnées pour se situer de part et d'autre du point isobestique pour l'oxygénation et la désoxygénation de l'hémoglobine sanguine ; ou
dans lequel la diode électroluminescente associée à chacun de la pluralité d'émetteurs est montée au sein d'un casque, le casque servant à porter la pluralité d'émetteurs et la pluralité de détecteurs en contact avec le cuir chevelu de l'utilisateur lorsqu'il est porté par l'utilisateur.

5. Appareil selon la revendication 1, dans lequel le dispositif de commande (110) est configuré de manière opérationnelle : pour activer des émetteurs et détecteurs sélectionnés pour générer des signaux associés à différents passages de déplacement du rayonnement infrarouge à travers le tissu cérébral ; ou pour traiter les signaux de passage superficiel pour déterminer un bruit de passage superficiel, le bruit de passage superficiel étant utilisé comme une base pour le filtrage du signal de passage en profondeur pour déterminer les changements de l'oxygénation sanguine au sein du tissu cérébral.

6. Appareil selon la revendication 5, dans lequel le dispositif de commande est configuré de manière opérationnelle pour traiter les signaux par :
l'alignement d'une phase de chacun des signaux de passage superficiel et des signaux de passage en profondeur sur la base d'une composante de processus physiologique dans les signaux ;
la réalisation d'une analyse en composantes principales sur les signaux de passage superficiel pour déterminer des composantes de contamination associées à des processus physiologiques autres que des changements de l'oxygénation sanguine au sein du tissu cérébral ; et
le retrait des composantes de contamination à partir des signaux de passage en profondeur pour fournir des signaux représentant des changements de l'oxygénation sanguine au sein du tissu cérébral desquels les effets d'autres processus physiologiques ont été filtrés.

7. Appareil selon la revendication 6, dans lequel la réalisation de l'analyse en composantes principales comprend :
le filtrage des signaux de passage superficiel pour séparer les signaux de passage superficiel en signaux à cycle lent associés à des processus physiologiques à cycle lent et en signaux à cycle rapide associés à des processus physiologiques à cycle rapide ; et
la réalisation d'une analyse en composantes principales sur chacun des signaux de passage superficiel, des signaux à cycle lent et des signaux à cycle rapide ; ou dans lequel le dispositif de commande est configuré de manière opérationnelle pour, avant la réalisation de l'analyse en composantes principales :
traiter les signaux de passage superficiel alignés en phase pour générer des signaux représentant l'oxygénation et la désoxygénation de l'hémoglobine sanguine ; et
prendre une dérivée première des signaux représentant l'oxygénation et la désoxygénation de l'hémoglobine sanguine.

8. Appareil selon la revendication 1, dans lequel :
(a) la longueur de chaque conduit de lumière (106) est entre environ 7 millimètres et 15 millimètres ; et dans lequel une longueur d'au moins environ 7 mm du conduit de lumière fait saillie vers l'extérieur à partir d'une surface du casque ; ou
(b) dans lequel la partie guide du conduit de lumière possède une forme généralement cylindrique et possède un diamètre sélectionné pour provoquer une réflexion interne totale d'un rayonnement infrarouge incident au niveau de surfaces internes de la partie guide.

9. Appareil selon la revendication 8, dans lequel une aire de section transversale de la partie guide est plus petite qu'une aire de section transversale de la surface de couplage et le conduit de lumière comprend en outre une transition effilée entre la surface de couplage et la partie guide et dans lequel un angle effilé de la transition effilée est sélectionné pour empêcher une fuite de rayonnement infrarouge à partir de la transition effilée, la transition effilée permettant en outre le montage du conduit de lumière sur l'émetteur ou le détecteur.

10. Appareil selon la revendication 1, dans lequel la surface de couplage du conduit de lumière est configurée pour être directement en contact avec une surface rayonnante de l'émetteur ou une surface de réception de rayonnement du détecteur pour le couplage d'un rayonnement infrarouge entre le conduit de lumière et le détecteur.

11. Appareil selon la revendication 1, dans lequel chacun de la pluralité de segments articulés (112, 114) est configuré de manière opérationnelle pour qu'un substrat de circuit y soit monté et dans lequel au moins un détecteur ou un émetteur est monté sur chaque substrat de circuit ; comprenant en outre une interconnexion souple (504) formant une interconnexion entre un dispositif de commande de casque (600) et la pluralité de substrats de circuit (210) ; et dans lequel l'interconnexion souple et la pluralité de substrats de circuit sont formés comme un substrat de circuit souple unitaire.

12. Appareil selon la revendication 1, dans lequel le dispositif de commande est configuré de manière opérationnelle pour surveiller le niveau de signal produit au niveau de chaque détecteur et pour commander un niveau d'un rayonnement infrarouge produit par l'émetteur sélectivement actionné pour conserver l'intensité au sein d'une plage de détection du détecteur.

13. Appareil selon la revendication 12, dans lequel le dispositif de commande est en outre configuré de manière opérationnelle pour générer des données d'affichage pour un affichage comme une interface utilisateur graphique, GUI, sur un écran en communication avec le dispositif de commande, la GUI incluant une représentation spatiale d'au moins l'un des émetteurs et détecteurs conjointement avec des informations d'affichage indiquant si l'intensité de signal est au sein de la plage de détection du détecteur associé ; ou dans lequel le dispositif de commande est configuré de manière opérationnelle pour interrompre la surveillance lorsque les signaux reçus en provenance des détecteurs ne respectent plus un critère de couplage indicatif du fait qu'une pluralité des émetteurs ou des détecteurs sont couplés au cuir chevelu de l'utilisateur.

14. Appareil selon la revendication 1, dans lequel l'au moins un capteur de couplage comprend au moins l'un parmi :
un capteur capacitif qui produit un signal indicatif d'une proximité de l'appareil avec le cuir chevelu ;
un capteur acoustique qui produit un signal en réponse à un niveau sonore ambiant ;
un capteur inertiel qui produit un signal indicatif d'un mouvement de l'appareil ; ou
l'un ou plusieurs des détecteurs, dans lequel une composante de lumière ambiante dans le signal produit par les un ou plusieurs détecteurs est indicative du fait que l'appareil est retiré du cuir chevelu et que le détecteur est soumis à un rayonnement de lumière ambiante.

15. Appareil selon la revendication 13, dans lequel le dispositif de commande est configuré pour traiter le signal reçu par au moins l'un des détecteurs pour extraire un signal de pouls cardiaque représentant un battement cardiaque détecté de l'utilisateur et pour surveiller le signal de pouls pour déterminer si un couplage entre les émetteurs et détecteurs et le cuir chevelu de l'utilisateur respecte un critère de couplage.

16. Appareil selon la revendication 15, dans lequel le dispositif de commande est configuré de manière opérationnelle : pour traiter les signaux par l'extraction d'une fréquence dominante à partir des signaux qui se situe au sein d'une plage de fréquences sur la base de la plage de fréquences de battement cardiaque attendue de l'utilisateur ; et/ou pour interrompre la surveillance lorsque les signaux de pouls cardiaque reçus en provenance des détecteurs ne respectent plus le critère de couplage.

17. Appareil selon la revendication 1, dans lequel le dispositif de commande est configuré de manière opérationnelle pour surveiller des variations temporelles de changements de l'oxygénation sanguine au sein du tissu cérébral dans une région sous-jacente à chaque détecteur et émetteur sélectivement actionné et pour générer des métriques de données représentant un degré d'activation cérébrale dans chaque région ; ou
dans lequel le dispositif de commande est en outre configuré de manière opérationnelle pour générer des données d'affichage pour un affichage comme une interface utilisateur graphique, GUI, sur un écran en communication avec le dispositif de commande, la GUI incluant une représentation de régions du corps de l'utilisateur qui correspondent à des régions du cerveau de l'utilisateur qui sont indiquées par les changements de l'oxygénation sanguine au sein du tissu cérébral devant être actionné ; ou
dans lequel le dispositif de commande comprend un circuit de processeur, le circuit de processeur incluant une unité de traitement graphique configurée de manière opérationnelle pour accélérer le traitement des signaux provenant de chacun des détecteurs pour faciliter une présentation en temps quasi réel des résultats à l'utilisateur.
